# EUROPEAN PATENT APPLICATION

(11) **EP 4 403 576 A1**
(43) Date of publication of application: **24.07.2024**
(21) Application number: 22870208.0
(22) Date of filing: 07.09.2022
(51) Int. Cl.: C07K 16/28, A61P 35/00, A61K 39/00

(54) **GLYCOSYLATED FC VARIANTS WITH IMPROVED SELECTIVE BINDING AFFINITY TO FC GAMMA RIIIA**

(30) Priority: 17.09.2021 KR 20210124554
(71) Applicant: Korea University Research and Business Foundation, Seoul 02841 (KR)
(72) Inventor: JUNG, Sang Taek, Seoul 06217 (KR); JO, Migyeong, Seoul 02717 (KR); KIM, Suyeon, Seoul 03428 (KR)
(74) Representative: Isarpatent
(86) International application number: PCT/KR2022/013476
(87) International publication number: WO 2023/043123

(57) **Abstract**

The present invention relates to a glycosylated Fc variant with improved selective binding affinity to FcγRIIIa. Novel human antibody Fc domain variants of the present invention have reduced binding affinity to FcγRIIIb, which is an immuno-inhibitory receptor, compared to antibodies approved as conventional antibody therapeutic agents, and have improved binding affinity to FcγRIIIa (an increase in the A/I ratio), which is an immuno-activating receptor, and thus have a significantly improved ability to induce ADCC, and have the effect of maximizing the immune action mechanism of therapeutic protein drugs, and thus can be effectively used as antibody drugs.

## Description

### [Technical Field]

The present invention relates to a glycosylated Fc variant with improved selective binding affinity to FcyRIIIa.

### [Background Art]

With the development of biotechnology such as genetic recombination and cell culture, research on the structure and function of proteins has been actively conducted around the world, which not only improves the understanding of life phenomena, but also plays a decisive role in identifying the pathogenic mechanisms of various diseases, thereby providing the way for effective disease diagnosis and treatment, and greatly contributing to improving the quality of life. In particular, since hybridoma technology, which produced monoclonal antibodies by fusing B cells and myeloma cells, was developed in 1975 (Kohler and Milstein, Nature, 256:495-497, 1975), research and development on immunotherapy using therapeutic antibodies have been actively conducted in clinical fields such as cancer, autoimmune disease, inflammation, cardiovascular disease, and infection.

An antibody provides a linkage between the humoral and cellular immune systems, and a Fab region of the antibody recognizes an antigen, whereas an Fc domain region binds to a receptor (Fc receptor or FcR) for an antibody (immunoglobulin) on a cell that is differentially expressed by all immunocompetent cells. An Fc receptor binding site on the Fc region of the antibody binds to a cell through the Fc region by binding to the Fc receptor (FcR) on the cell, and the antibody binds to the Fc receptor on the cell surface to trigger a variety of important biological responses, including control of phagocytosis and destruction of antibody-coated particles, removal of immune complexes, lysis of antibody-coated target cells by killing cells (antibody-dependent cell-mediated cytotoxicity, or ADCC), release of inflammatory mediators, placental transfer and immunoglobulin production (Deo, Y.M. et al., Immunol. Today 18(3):127-135 (1997)). As such, the Fc domain plays a critical role in the collection of immune cells and ADCC and antibody dependent cellular phagocytosis (ADCP). In particular, the ADCC and ADCP functions, which are the effector functions of the antibody, depend on interaction with Fc receptors present on the surfaces of many cells. Human Fc receptors are classified into five types, and the type of immune cell collected is determined depending on which Fc receptor the antibody binds to. Accordingly, attempts to modify antibodies to collect specific cells may be very important in the field of treatment.

A therapeutic antibody shows very high specificity for targets compared to conventional small molecule drugs, has low biotoxicity and few side effects, and has an excellent blood half-life of about 3 weeks, and thus is considered as one of the most effective cancer therapy methods. In fact, large pharmaceutical companies and research institutes around the world are accelerating the research and development of therapeutic antibodies that specifically bind to cancer cells, including cancer-causing factors, to effectively remove the cancer cells. Companies for developing therapeutic antibody drugs mainly consist of pharmaceutical companies such as Roche, Amgen, Johnson & Johnson, Abbott, and BMS. Particularly, representative products of Roche include Herceptin, Avastin, Rituxan, and the like for anti-cancer treatment, and these three therapeutic antibodies not only achieved large profits, achieving sales of approximately $19.5 billion in the global market in 2012, but are also leading the antibody pharmaceutical market of the world. Johnson & Johnson, which developed Remicade, is also growing rapidly in the global antibody market due to increased sales, and pharmaceutical companies such as Abbott and BMS are also known to have many therapeutic antibodies in the final stages of development. As a result, biopharmaceuticals containing therapeutic antibodies that are specific for disease targets and have low side effects are rapidly replaced in the global pharmaceutical market, where small molecule drugs used to dominate.

Currently, the action of antibodies as pharmaceuticals has a direct method by binding to an antigen to inhibit the action of the antigen, and an indirect antigen removal method by effector cells (natural killer cells, macrophages, etc.) having an Fc gamma region and an Fc gamma receptor (FCGR) of the antibody binding to the antigen. In the case of an antibody therapeutic agent that is being developed recently, the effect of the drug is increased through an ADCC mechanism in which the effector cell recognizes the Fc gamma region of the antibody and attacks and removes the antigen while preventing the action of the antigen due to binding to the antigen. In a recent ADCC-related study, it has been found that the binding affinity of the Fc gamma and Fc gamma receptor is affected depending on a genotype of the Fc gamma region receptor. Many studies have reported that the efficiency of the antibody-based therapeutic agent varies depending on the diversity of amino acid sequences of amino acid at position 131 of FCGR2A protein (FcγRIIIa) and amino acid at position 158 of FCGR3A protein, which are Fc gamma receptors of a patient. For example, in the case of Herceptin (trastuzumab), which is a breast cancer therapeutic agent, there was reported in 2008 by Musolino, et al. a study that antibody-dependent cytotoxicity was increased with a significant difference in FCGR2A 131 H/H or FCGR3A 158 V/V genotype in preclinical studies. However, the binding affinity to a specific Fc receptor is increased by modifying an Fc region of a mammalian antibody, but the binding affinity to other Fc receptors is also maintained, so that there is a problem that undesirable immune responses are still maintained. Four of five major FcyRs in humans induce immuno-activating or inflammatory responses, and FcyRIIb induces immuno-inhibitory or anti-inflammatory responses. Most naturally occurring antibody or recombinant glycosylated antibodies bind to activating and inhibitory Fc receptors. The immune cells that most strongly induce the cancer cell death effect of therapeutic IgG antibodies through ADCC are natural killer cells (NK cells), and the NK cells express FcyRIIIa on the surface, but do not express FcyRI and FcyRIIa, FcyRIIb, and FcyRIIIb unlike other immune cells (e.g., monocytes, macrophages, dendritic cells, etc.), and thus, in order to maximize the cancer cell death mechanism, it is required to improve the affinity to FcyRIIIa expressed on the surface of the NK cells through optimization of the Fc region of the IgG antibody. In addition, considering the complex immune response of not only NK cells but also various immune cells present in the blood, the higher a ratio (A/I ratio) of the ability (A) of the Fc domain of an antibody to bind to activating FcyR and the ability (I) to bind to inhibitory FcyRIIb, the higher the ability to induce ADCC, and thus, it is very urgent to selectively increase the binding affinity to the activating receptor compared to the binding affinity to FcyRIIb, which is an inhibitory receptor (Boruchov et al, J Clin Invest, 115(10):2914-23, 2005). However, due to a problem that FcyRIIb has 96% homology with activating FcyR, efforts to increase the A/I ratio by introducing genetic mutations into glycosylated antibodies have not yielded significant results.

### [Disclosure]

### [Technical Problem]

An object of the present invention is to provide novel human antibody Fc domain variants.

Further, another object of the present invention is to provide a glycosylated antibody specific for a specific Fc gamma receptor or an immunologically active fragment thereof.

Further, yet another object of the present invention is to provide a nucleic acid molecule encoding the Fc domain variant, or the antibody or the immunologically active fragment thereof, a vector including the nucleic acid molecule, and a host cell including the vector.

Further, still another object of the present invention is to provide a pharmaceutical composition for preventing or treating cancer.

Further, still another object of the present invention is to provide a method for preparing a human antibody Fc domain variant.

Further, still another object of the present invention is to provide a method for preparing a glycosylated antibody with improved selective binding affinity to FcyRIIIa.

Further, still another object of the present invention is to provide a use of an antibody or an immunologically active fragment thereof to be used for preparation of an antibody therapeutic agent.

Further, still another object of the present invention is to provide a use of an antibody or an immunologically active fragment thereof for preventing or treating cancer.

Further, still another object of the present invention is to provide a method for treating cancer including administering an antibody or an immunologically active fragment thereof in a pharmaceutically effective amount to a subject suffering from cancer.

### [Technical Solution]

An aspect of the present invention provides novel human antibody Fc domain variants with improved selective binding affinity to a specific Fc gamma receptor.

Further, another aspect of the present invention provides a glycosylated antibody including the novel human antibody Fc domain variant or an immunologically active fragment thereof.

Further, yet another aspect of the present invention provides a nucleic acid molecule encoding the Fc domain variant, or the antibody or the immunologically active fragment thereof, a vector including the nucleic acid molecule, and a host cell including the vector.

Further, still another aspect of the present invention provides a pharmaceutical composition for preventing or treating cancer including the Fc domain variant, or the antibody or the immunologically active fragment thereof as an active ingredient.

Further, still another aspect of the present invention provides a method for preparing a human antibody Fc domain variant.

Further, still another aspect of the present invention provides a method for preparing a glycosylated antibody with improved selective binding affinity to FcyRIIIa.

Further, still another aspect of the present invention provides a use of an antibody of the present invention or an immunologically active fragment thereof to be used for preparation of an antibody therapeutic agent.

Further, still another aspect of the present invention provides a use of an antibody of the present invention or an immunologically active fragment thereof for preventing or treating cancer.

Further, still another aspect of the present invention provides a method for treating cancer including administering an antibody of the present invention or an immunologically active fragment thereof in a pharmaceutically effective amount to a subject suffering from cancer.

### [Advantageous Effects]

According to the present invention, novel human antibody Fc domain variants have reduced binding affinity to FcyRIIb, which is an immuno-inhibitory receptor, compared to antibodies approved as conventional antibody therapeutic agents, and have improved binding affinity to FcyRIIIa (an increase in the A/I ratio), which is an immuno-activating receptor, and thus have a significantly improved ability to induce ADCC, and have the effect of maximizing the immune action mechanism of therapeutic protein drugs, and thus may be effectively used as antibody drugs.

### [Description of Drawings]

FIG. 1 is a diagram of confirming four trastuzumab Fc variants (XMa, XMt, MM and XMM) consisting of simple combinations of known mutations (A) and FcyRIIb-GST protein (B) through SDS-PAGE gel after high-purity purification.
FIG. 2 is a diagram illustrating ELISA results of analyzing binding affinity to FcyRIIb of purified trastuzumab Fc variants (Trastuzumab-XMa, XMt, MM and XMM).
FIG. 3 is a schematic diagram of a mammalian cell display technology for glycosylated Fc.
FIG. 4 is a diagram of confirming high-purity purified tetrameric FcyRIIIa-158V-streptavidin, tetrameric FcyRIIIa-158F-streptavidin, tetrameric FcyRIIb-streptavidin, FcγRIIIa-158V-GST and FcyRIIIa-158F-GST through SDS-PAGE gel.
FIG. 5 is a diagram of analyzing binding activity of fluorescent-labeled tetrameric FcyRIIIa-Alexa488, tetrameric FcyRIIIa-Alexa647, and Protein A-FITC with wild-type Fc or Fc-T299L expressed in CHO cells.
FIG. 6 is a schematic diagram illustrating a shuffle library for glycosylated Fc engineering.
FIG. 7 is a schematic diagram of glycosylated Fc engineering using a CHO cell display and a diagram showing the gene logos of mutations of glycosylated Fc variants obtained as a result of screening.
FIG. 8 is a diagram illustrating ELISA results of analyzing binding affinity to FcyRIIIa using an Expi293F cell culture solution cultured by cloning glycosylated Fc variants into a trastuzumab heavy chain gene as a model antibody and transfecting an expression vector and mutation sequences of discovered Fc variants.
FIG. 9 is a diagram of confirming trastuzumab Fc variants (PS101, PS102, PS105, PS106, PS107, PS205, PS207, PS301, PS303 and PS305) including glycosylated Fc variants with high binding affinity to FcyRIIIa through SDS-PAGE gel after purification.
FIGS. 10 and 11 are diagrams of analyzing binding affinity to FcyRIIIa-158V, FcyRIIIa-158F and FcyRIIb of selected glycosylated trastuzumab Fc variants (PS101, PS102, PS105, PS106, PS107, PS205, PS207, PS301, PS303, and PS305) through ELISA.
FIG. 12 is a diagram of comparatively analyzing binding tendency to FcyRIIIa-158V, FcyRIIb, FcyRIIIa-158F or FcyRIIb of trastuzumab Fc variants of the present invention (PS101, PS102, PS105, PS106, PS107, PS205, PS207, PS301, PS303, and PS305) and trastuzumab Fc variants DE and VLPLL into which Fcs of tafasitamab and margetuximab, glycosylated antibodies previously approved by the US FDA as therapeutic agents for B-cell lymphoma and breast cancer, are introduced, respectively.
FIG. 13 is a diagram illustrating results of analyzing HER2 binding of wild-type trastuzumab (TRZ), positive control trastuzumab Fc variants (TRZ-DE and TRZ-VLPLL) and trastuzumab Fc variants (TRZ-PS101, TRZ-PS102 and TRZ-PS107).
TRZ: trastuzumab.
FIG. 14 is a diagram illustrating results of analyzing binding affinity to FcyRIIIa (CHO-FcyRIIIa-158V and CHO-FcyRIIIa-158F) membrane anchored on CHO cells of wild-type trastuzumab (TRZ), positive control trastuzumab Fc variants (TRZ-DE and TRZ-VLPLL) and trastuzumab Fc variants (TRZ-PS101, TRZ-PS102 and TRZ-PS107).
FIG. 15 is a diagram illustrating results of ADCC analysis of wild-type trastuzumab (TRZ), positive control trastuzumab Fc variants (TRZ-DE and TRZ-VLPLL) and trastuzumab Fc variants (TRZ-PS101, TRZ-PS102 and TRZ-PS107), when a ratio of E :T (Effector cell : target cell) is 2 : 1.
   A: Real-time % of cytotoxicity analysis graph; and
   B: Endpoint % of cytotoxicity analysis graph based on real-time analysis graph.
FIG. 16 is a diagram illustrating results of ADCC analysis of wild-type trastuzumab (TRZ), positive control trastuzumab Fc variants (TRZ-DE and TRZ-VLPLL) and trastuzumab Fc variants (TRZ-PS101, TRZ-PS102 and TRZ-PS107), when a ratio of E : T is 5 : 1.
   A: Real-time % of cytotoxicity analysis graph; and
   B: Endpoint % of cytotoxicity analysis graph based on real-time analysis graph.
FIG. 17 is a heat map showing results of IEDB-based *in silico* immunogenicity analysis of wild-type Fc and Fc variants of the present invention (PS101, PS102, and PS107).
FIG. 18 is a diagram showing results of analysis by SDS-PAGE after preparing mouse FcyRIV-GST and cynomolgus monkey FcyRIII-GST.
FIG. 19 is a diagram illustrating results of analyzing binding affinity to mouse FcyRIV and cynomolgus monkey FcyRIII of wild-type trastuzumab (TRZ), positive control trastuzumab Fc variants (TRZ-DE and TRZ-VLPLL) and trastuzumab Fc variants (TRZ-PS101, TRZ-PS102 and TRZ-PS107).
FIG. 20 is a diagram illustrating results of analysis by SDS-PAGE after expressing and purifying wild-type cetuximab (CTX-WT), positive control cetuximab Fc variants (CTX-DE and CTX-VLPLL) and cetuximab Fc variants (CTX-PS101, CTX-PS102 and CTX-PS107); and wild-type rituximab (RTX-WT), positive control rituximab Fc variants (RTX-DE and RTX-VLPLL) and rituximab Fc variants (RTX-PS101, RTX-PS102 and RTX-PS107).
FIG. 21 is a diagram illustrating results of analyzing binding affinity to human FcyRIIIa-158V, human FcyRIIIa-158F and human FcyRIIb of wild-type cetuximab (CTX-WT), positive control cetuximab Fc variants (CTX-DE and CTX-VLPLL) and cetuximab Fc variants (CTX-PS101, CTX-PS102 and CTX-PS107).
FIG. 22 is a diagram illustrating results of analyzing binding affinity to mouse FcyRIV and cynomolgus monkey FcyRIII of wild-type cetuximab (CTX-WT), positive control cetuximab Fc variants (CTX-DE and CTX-VLPLL) and cetuximab Fc variants (CTX-PS101, CTX-PS102 and CTX-PS107).
FIG. 23 is a diagram illustrating results of analyzing binding affinity to human FcyRIIIa-158V, human FcyRIIIa-158F and human FcyRIIb of wild-type rituximab (RTX-WT), positive control rituximab Fc variants (RTX-DE and RTX-VLPLL) and rituximab Fc variants (RTX-PS101, RTX-PS102 and RTX-PS107).
FIG. 24 is a diagram illustrating results of analyzing binding affinity to mouse FcyRIV and cynomolgus monkey FcyRIII of wild-type rituximab (RTX), positive control rituximab Fc variants (RTX-DE and RTX-VLPLL) and rituximab Fc variants (RTX-PS101, RTX-PS102 and RTX-PS107).

### [Best Mode of the Invention]

Hereinafter, embodiments of the present invention will be described in detail with reference to the accompanying drawings. However, the following embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present invention are possible within the description of claims to be described below and the equivalent scope interpreted therefrom.

Further, terminologies used in the present specification are terminologies used to properly express preferred examples of the present invention, which may vary according to a user, an operator's intention, or customs in the art to which the present invention pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

All technical terms used in the present invention, unless otherwise defined, have the meaning as commonly understood by those skilled in the related art of the present invention. In addition, although preferred methods and samples are described herein, similar or equivalent methods and samples thereto are also included in the scope of the present invention. The contents of all publications disclosed as references in this specification are incorporated in the present invention.

Throughout the present specification, general one-letter or three-letter codes for naturally existing amino acids are used, and generally allowed three-letter codes for other amino acids, such as α-aminoisobutyric acid (Aib) and N-methylglycine (Sar) are also used. The amino acids mentioned herein as abbreviations are also described as follows according to the IUPAC-IUB nomenclature.

Alanine: A, Arginine: R, Asparagine: N, Aspartic acid: D, Cysteine: C, Glutamic acid: E, Glutamine: Q, Glycine: G, Histidine: H, Isoleucine: I, Leucine: L, Lysine: K, Methionine: M, Phenylalanine: F, Proline: P, Serine: S, Threonine: T, Tryptophan: W, Tyrosine: Y, and Valine: V.

In an aspect, the present invention relates to a human antibody Fc domain variant in which one or more amino acids selected from the group consisting of amino acids at positions 222, 224, 239, 243, 247, 252, 292, 300, 303, 305, 330, 332, 339, 356, 361, 387, 396, 405 and 415 numbered according to a Kabat numbering system in a wide-type human antibody Fc domain are substituted with sequences different from wild-type amino acids.

In an embodiment, in the human antibody Fc domain variant of the present invention, one or more amino acids selected from the group consisting of amino acids at positions 224, 243, 247, 292, 300, 303, 305, 330, 332, 339, 356 and 387 may be substituted with sequences different from wild-type amino acids.

In an embodiment, the human antibody Fc domain variant of the present invention may include one or more amino acid substitutions selected from the group consisting of K222N, H224R, S239D, F243L, P247I, M252V, R292P, Y300L, V303I, V305I, A330L, I332E, A339Q, D356G, N361D, P387Q, P396L, F405L and S415G.

In an embodiment, the human antibody Fc domain variant may include one or more amino acid substitutions selected from the group consisting of H224R, F243L, P247I, R292P, Y300L, V303I, V305I, A330L, I332E, A339Q, D356G and P387Q.

In an embodiment, the Fc domain variant of the present invention may include any one selected from the group consisting of amino acid sequences represented by SEQ ID NOs: 22 to 35.

In an embodiment, the human antibody Fc domain variant may be PS101 including amino acid substitutions of F243L, P247I, R292P, Y300L, V305I, A330L, I332E, A339Q and P387Q.

In an embodiment, the human antibody Fc domain variant may be PS102 including amino acid substitutions of H224R, F243L, P247I, R292P, Y300L, V303I, V305I, A330L, I332E, A339Q and P387Q.

In an embodiment, the human antibody Fc domain variant may be PS107 including amino acid substitutions of F243L, P247I, R292P, Y300L, V305I, A330L, I332E, A339Q, D356G and P387Q.

In an embodiment, the human antibody Fc domain variant may be PS106 including amino acid substitutions of F243L, P247I, M252V, R292P, Y300L, V305I, A330L, I332E, A339Q, D356G and P387Q.

In an embodiment, the human antibody Fc domain variant may be PS205 including amino acid substitutions of F243L, P247I, R292P, Y300L, A330L, I332E and P396L.

In an embodiment, the human antibody Fc domain variant may be PS301 including amino acid substitutions of K222N, F243L, P247I, R292P, Y300L, V305I, A330L and I332E.

In an embodiment, the human antibody Fc domain variant may be PS303 including amino acid substitutions of S239D, P247I, Y300L, V305I, A330L, I332E, A339Q, N361D and P387Q.

In an embodiment, the human antibody Fc domain variant may be PS305 including amino acid substitutions of S239D, R292P, Y300L, A330L, A339Q, P387Q and F405L.

In an embodiment, the human antibody Fc domain variant may be PS105 including amino acid substitutions of R292P, Y300L, V305I, A330L, I332E, A339Q and P387Q.

In an embodiment, the human antibody Fc domain variant may be PS207 including amino acid substitutions of F243L, P247I, R292P, A330L, I332E, A339Q, P396L, and S415G.

In an embodiment, the Fc domain variant of the present invention may have improved binding affinity to FcyRIIIa and reduced binding affinity to FcyRIIb compared to the wild-type Fc domain.

In an embodiment, the Fc domain variant of the present invention may specifically bind to a human Fcy receptor, a mouse Fcy receptor, or a monkey Fcy receptor, the human Fcyreceptor may be FcyRIIIa-158V or FcyRIIIa-158F, the mouse Fcy receptor may be FcyIV, and the monkey Fcy receptor may be cynomologous monkey FcyRIII.

In an embodiment, the Fc domain variant of the present invention may have improved ability to induce antibody-dependent cell-mediated cytotoxicity (ADCC).

In an embodiment, the human antibody (immunoglobulin) may be IgA, IgM, IgE, IgD or IgG, or a variant thereof, and may be IgG1, IgG2, IgG3 or IgG4, preferably an anti-human epidermal growth factor receptor 2 (HER2) antibody, an anti-epidermal growth factor receptor (EGFR) antibody, or an anti-CD20 antibody, and more preferably trastuzumab, cetuximab or rituximab. Papain degradation of the antibody forms two Fab domains and one Fc domain, and in a human IgG molecule, the Fc region is generated by papain degradation of the N-terminus at Cys 226 (Deisenhofer, Biochemistry 20: 2361-2370, 1981).

In an embodiment, the trastuzumab may include a light chain including an amino acid sequence represented by SEQ ID NO: 51, and a heavy chain including an amino acid sequence represented by SEQ ID NO: 52, and trastuzumab TRZ-PS101, TRZ-PS102 or TRZ-PS107 including the Fc variant of the present invention may include a heavy chain including an amino acid sequence represented by SEQ ID NO: 53, 54 or 55.

In an embodiment, the cetuximab may include a light chain including an amino acid sequence represented by SEQ ID NO: 56, and a heavy chain including an amino acid sequence represented by SEQ ID NO: 57, and cetuximab CTX-PS101, CTX-PS102 or CTX-PS107 including the Fc variant of the present invention may include a heavy chain including an amino acid sequence represented by SEQ ID NO: 58, 59 or 60.

In an embodiment, the rituximab may include a light chain including an amino acid sequence represented by SEQ ID NO: 61, and a heavy chain including an amino acid sequence represented by SEQ ID NO: 62, and rituximab RTX-PS101, RTX-PS102 or RTX-PS107 including the Fc variant of the present invention may include a heavy chain including an amino acid sequence represented by SEQ ID NO: 63, 64 or 65.

In an embodiment, the wild-type human antibody Fc domain may include an amino acid sequence represented by SEQ ID NO: 21 and may be encoded with a nucleic acid molecule represented by SEQ ID NO: 36.

In the present invention, variants containing amino acid mutations in the human antibody Fc region of the present invention are defined according to the amino acid modifications constituting an Fc region of a parent antibody, and conventional antibody numbering is according to the EU index by Kabat (Kabat et al., Sequence of proteins of immunological interest, 5th Ed., United States Public Health Service, National Institutes of Health, Bethesda, 1991).

As used in the present invention, the term "Fc domain variant" may be used interchangeably with the "Fc variant".

As used in the present invention, the term "wild-type polypeptide" refers to an unmodified polypeptide that is modified later to produce a derivative. The wild-type polypeptide may be a polypeptide found in nature, or a derivative or manipulation of the polypeptide found in nature. The wild-type polypeptide may refer to a polypeptide itself, a composition containing the wild-type polypeptide, or an amino acid sequence encoding the same. Accordingly, as used in the present invention, the term "wild-type antibody" refers to an unmodified antibody polypeptide in which amino acid residues are modified to produce a derivative. Interchangeably with the term, the "parent antibody" may be used to refer to an unmodified antibody polypeptide into which amino acid modifications are introduced to produce a derivative.

As used in the present invention, the term "amino acid modification/mutation" refers to substitution, insertion and/or deletion, preferably substitution of amino acids in a polypeptide sequence. As used in the present invention, the term "amino acid substitution" or "substitution" means that an amino acid at a specific position in the polypeptide sequence of the wild-type human antibody Fc domain is replaced with another amino acid. For example, an Fc variant including T299A substitution means that threonine, an amino acid residue at position 299 in the amino acid sequence of the Fc domain of the wild-type antibody, is replaced with alanine.

As used in the present invention, the term "Fc variant" means including a modification of one or more amino acid residues compared to the wild-type antibody Fc domain.

The Fc variant of the present invention includes one or more amino acid modifications compared to the wild-type antibody Fc domain (region or fragment), resulting in a difference in amino acid sequence. The amino acid sequence of the Fc variant according to the present invention is substantially homologous to the amino acid sequence of the wild-type antibody Fc domain. For example, the amino acid sequence of the Fc variant according to the present invention has about 80% or more, preferably about 90% or more, and most preferably about 95% or more homology compared to the amino acid sequence of the wild-type antibody Fc domain. Amino acid modifications may be performed genetically using molecular biological methods, or also performed using enzymatic or chemical methods.

The Fc variants of the present invention may be prepared by any method known in the art. In an example, the Fc variant of the human antibody according to the present invention is used to form a nucleic acid in which a polypeptide sequence containing a specific amino acid modification is encoded and then, if desired, cloned into a host cell, expressed, and assayed. Various methods therefor are described in the literature (Molecular Cloning-A Laboratory Manual, 3rd Ed., Maniatis, Cold Spring Harbor Laboratory Press, New York, 2001; Current Protocols in Molecular Biology, John Wiley & Sons).

The nucleic acid encoding the Fc variant according to the present invention may be inserted into an expression vector for protein expression. The expression vector generally includes a protein operably linked, i.e., functionally related to a control or regulatory sequence, a selectable marker, an optional fusion partner, and/or an additional element. Under appropriate conditions, the Fc variant according to the present invention may be produced by a method of inducing the protein expression by culturing a host cell transformed with nucleic acid, preferably a nucleic acid-containing expression vector encoding the Fc variant according to the present invention. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, the Fc variant according to the present invention is produced using E. coli, which has low production cost and high industrial value, as a host cell.

Accordingly, the scope of the present invention includes a method for preparing an Fc variant including culturing a host cell into which a nucleic acid encoding an Fc variant has been introduced, under conditions suitable for protein expression; and purifying or isolating the Fc variant expressed from the host cell.

In an aspect, the present invention relates to an antibody including the Fc domain variant of the present invention or an immunologically active fragment thereof.

In an embodiment, the antibody may be a glycosylated antibody.

In an embodiment, the antibody of the present invention may include a heavy chain constant region domain 2(C_{H}2) including any one selected from the group consisting of amino acid sequences represented by SEQ ID NOs: 1 to 13 and a heavy chain constant region domain 3(C_{H}3) including any one selected from the group consisting of amino acid sequences represented by SEQ ID NOs: 14 to 20, and more preferably a heavy chain constant region domain 2(C_{H}2) including an amino acid sequence represented by SEQ ID NO: 6 or 7 and a heavy chain constant region domain 3(C_{H}3) including an amino acid sequence represented by SEQ ID NO: 18 or 19.

In an embodiment, the immunologically active fragment may be any one selected from the group consisting of Fab, Fd, Fab', dAb, F(ab'), F(ab')₂, a single chain fragment variable (scFv), Fv, a single chain antibody, a Fv dimmer, a complementarity determining region fragment, a humanized antibody, a chimeric antibody, and a diabody.

In an embodiment, the antibody including the Fc domain variant of the present invention, or the immunologically active fragment thereof may increase an effector action, and has improved binding affinity to FcyRIIIa and reduced binding affinity to FcyRIIb compared to a wild-type Fc domain to have high FcyRIIIa binding selectivity and a high A/I ratio, thereby increasing antibody dependent cellular cytotoxicity (ADCC).

In the present invention, the A/I ratio is a ratio (A/I ratio) between the ability of the Fc domain of the antibody to bind to active FcyR (A) and the ability (I) to bind to inhibitory FcyRIIb, and as the A/I ratio is increased, excellent ability to induce ADCC is shown, so that it is important to selectively increase the binding affinity of the active receptor compared to the binding affinity of FcyRIIb, which is an inhibitory receptor.

Generally, a glycosylated antibody expressed in mammals has a protein structure stabilized by sugar chains modified in the Fc region so that the antibody may bind to the Fc receptor, but has binding affinity to all FcyRs, so that there has been a problem of inhibition occurring simultaneously with activation of the immune response. On the contrary, aglycosylated antibodies produced in bacteria have the problem of not being able to bind to Fc receptors to exhibit no ADCC function because there is no hydrocarbon chain bound to the Fc region. However, the antibody of the present invention is an 'aglycosylated' antibody or an immunologically active fragment thereof and thus has the effect of controlling the immune response by selectively improving binding affinity to FcyR.

The antibody may be isolated or purified by various methods known in the art. A standard purification method includes chromatographic techniques, electrophoresis, immunology, precipitation, dialysis, filtration, concentration, and chromatofocusing techniques. As known in the art, various natural proteins such as bacterial proteins A, G, and L are bound to the antibody and these proteins may be used for purification. Often, purification by specific fusion partners may be possible.

The antibody includes functional fragments of an antibody molecule as well as a whole antibody form. The whole antibody has a structure having two full-length light chains and two full-length heavy chains, and each light chain is linked to the heavy chain by disulfide bonds. The functional fragment of the antibody molecule refers to a fragment having an antigen-binding function. Examples of the antibody fragment include (i) a Fab fragment consisting of a light chain variable region VL, a heavy chain variable region VH, a light chain constant region CL, and a first heavy chain constant region CH1; (ii) a Fd fragment consisting of VH and CH1 domains; (iii) an Fv fragment consisting of VL and VH domains of a single antibody; (iv) a dAb fragment consisting of a VH domain (Ward ES et al., Nature 341:544-546 (1989)); (v) an isolated CDR region; (vi) a F(ab')2 fragment, which is a bivalent fragment including two linked Fab fragments; (vii) a single-chain Fv molecule (scFv) in which the VH domain and the VL domain are linked to each other by a peptide linker to form an antigen-binding domain; (viii) a bispecific single-chain Fv dimer (PCT/US92/09965); and (ix) a diabody which is a multivalent or multispecific fragment produced by gene fusion (WO94/13804).

The antibody of the present invention or the immunologically active fragment thereof may be selected from the group consisting of animal-derived antibodies, chimeric antibodies, humanized antibodies, human antibodies, and immunologically active fragments thereof. The antibody may be produced recombinantly or synthetically.

The antibody or the immunologically active fragment thereof may be isolated from a living body (not present in the living body) or may non-naturally occur, for example, may be synthetically or recombinantly produced.

As used in the present invention, the "antibody" refers to a substance produced by stimulation of an antigen in the immune system, and the type thereof is not particularly limited, and may be obtained naturally or non-naturally (e.g., synthetically or recombinantly). The antibody is advantageous for mass expression and production because of being very stable in vivo as well as in vitro and having a long half-life. In addition, since the antibody essentially has a dimer structure, avidity is very high. An intact antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and subclasses include gamma 1 (γ1), gamma 2 (γ2), gamma 3 (γ3), gamma 4 (y4), alpha 1 (α1) and alpha 2 (α2). The light chain constant region has kappa (κ) and lambda (λ) types.

As used in the present invention, the term "heavy chain" is interpreted as meaning including all of a full-length heavy chain including a variable region domain V_{H} including an amino acid sequence having a variable region sequence enough to impart specificity to an antigen, three constant region domains C_{H}1, C_{H}2 and C_{H}3 and hinges, and fragments thereof. In addition, the term "light chain" is interpreted as meaning including all of a full-length light chain including a variable region domain V_{L} including an amino acid sequence having a variable region sequence enough to impart specificity to an antigen and a constant region domain C_{L}, and fragments thereof.

As used in the present invention, the term "Fc domain", "Fc fragment", or "Fc region" forms an antibody with a Fab domain/fragment, and the Fab domain/fragment consists of a light chain variable region V_{L} and a heavy chain variable region V_{H}, a light chain constant region C_{L}, and a first heavy chain constant region C_{H}1, and the Fc domain/fragment consists of a second constant region C_{H}2 and a third constant region C_{H}3 of the heavy chain.

In an aspect, the present invention relates to a nucleic acid molecule encoding an Fc domain variant of the present invention, an antibody including the Fc domain variant, or an immunologically active fragment thereof.

In an embodiment, the nucleic acid molecule encoding the Fc variant of the present invention may include any one selected from the group consisting of 37 to 50 base sequences.

In an aspect, the present invention relates to a vector including the nucleic acid molecule and a host cell including the vector.

The nucleic acid molecule of the present invention may be isolated or recombinant, and includes not only DNA and RNA in single-stranded and double-stranded forms, but also complementary sequences corresponding thereto. The isolated nucleic acid is a nucleic acid that is isolated from surrounding genetic sequences present in the genome of a subject from which the nucleic acid is isolated, in the case of a nucleic acid isolated from a naturally occurring source. In the case of a nucleic acid synthesized enzymatically or chemically from a template, such as a PCR product, a cDNA molecule, or an oligonucleotide, the nucleic acid produced from such a procedure may be understood as an isolated nucleic acid molecule. The isolated nucleic acid molecule refers to a nucleic acid molecule in the form of a separate fragment or as a component of a larger nucleic acid construct. The nucleic acid is operably linked when disposed in a functional relationship with another nucleic acid sequence. For example, DNA of a pre-sequence or secretory leader is expressed as a preprotein in the form before the polypeptide is secreted to be operably linked to DNA of the polypeptide, and a promoter or enhancer is operably linked to a coding sequence when affecting the transcription of the polypeptide sequence, or a ribosome binding domain is operably linked to a coding sequence when disposed to promote the translation. In general, the operably linked means that DNA sequences to be linked are located contiguously, and that the secretory leader exists contiguously in the same leading frame. However, the enhancer needs not to be contiguously located. The linkage is achieved by ligation at a convenient restriction enzyme site. When there is no site, synthetic oligonucleotide adapters or linkers are used according to conventional methods.

In the isolated nucleic acid molecule encoding the Fc domain variant of the present invention, the antibody including the Fc domain variant or the immunologically active fragment thereof, due to the degeneracy of codons or in consideration of codons preferred in an organism in which the nucleic acid molecule is to be expressed, it will be understood well to those skilled in the art that various modifications may be made in a coding region within a range without changing the amino acid sequence of the Fc domain variant expressed from the coding region, the antibody including the Fc domain variant or the immunologically active fragment thereof, various modifications or changes may be made within a range without affecting the expression of the gene even in parts other than the coding region, and these modified genes are also included within the scope of the present invention. That is, as long as the nucleic acid molecule of the present invention encodes a protein having equivalent activity thereto, one or more nucleobases may be mutated by substitution, deletion, insertion, or a combination thereof, which are also included in the scope of the present invention. The sequence of such a nucleic acid molecule may be single- or double-stranded, and may be a DNA molecule or an RNA (mRNA) molecule.

The isolated nucleic acid molecule encoding the Fc domain variant of the present invention, the antibody including the Fc domain variant or the immunologically active fragment thereof may be inserted into an expression vector for protein expression. The expression vector generally includes a protein operably linked, i.e., functionally related to a control or regulatory sequence, a selectable marker, an optional fusion partner, and/or an additional element. In appropriate conditions, the Fc domain variant of the present invention, the antibody including the Fc domain variant or the immunologically active fragment thereof may be produced by a method for inducing the protein expression by culturing a host cell transformed with a nucleic acid, preferably an expression vector containing an isolated nucleic acid molecule encoding the Fc domain variant of the present invention, the antibody including the Fc domain variant or the immunologically active fragment thereof. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, it is possible to produce E. coli, which has high industrial value due to low production cost, as a host cell.

The vector of the present invention may include a plasmid vector, a cosmid vector, a bacteriophage vector, a viral vector, etc., but is not limited thereto. The suitable vector includes a signal sequence or a leader sequence for membrane targeting or secretion in addition to expression regulatory elements such as a promoter, an operator, an initiation codon, a termination codon, a polyadenylation signal, and an enhancer and may be variously produced according to a purpose. The promoter of the vector may be constitutive or inductive. The signal sequence may use a PhoA signal sequence, an OmpA signal sequence, etc. in the case of Escherichia sp. bacteria as a host, an α-amylase signal sequence, a subtilisin signal sequence, etc. in the case of Bacillus sp. bacteria as a host, an MFα signal sequence, a SUC2 signal sequence, etc. in the case of yeast as a host, and an insulin signal sequence, an α-interferon signal sequence, an antibody molecule signal sequence, etc. in the case of an animal cell as a host, but is not limited thereto. Further, the vector may include a selective marker for selecting a host cell including a vector and a replicable expression vector includes a replication origin.

As used in the present invention, the term "vector" refers to a vehicle into which a nucleic acid sequence may be inserted for introduction into a cell capable of replicating the nucleic acid sequence. The nucleic acid sequence may be exogenous or heterologous. The vector may include plasmids, cosmids, and viruses (e.g., bacteriophages), but is not limited thereto. Those skilled in the art may construct vectors by standard recombinant techniques (Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994, etc.).

In an embodiment, when constructing the vector, an expression regulatory sequence such as a promoter, a terminator, and an enhancer, a sequence for membrane targeting or secretion, etc. are appropriately selected according to a type of host cell to produce the Fc domain variant, the antibody including the Fc domain variant or the immunologically active fragment thereof and may be variously combined depending on a purpose.

As used in the present invention, the term "expression vector" refers to a vector including a nucleic acid sequence encoding at least a portion of a gene product to be transcribed. In some cases, the RNA molecule is then translated into a protein, a polypeptide, or a peptide. The expression vector may include various regulatory sequences. In addition to regulatory sequences that regulate transcription and translation, vectors and expression vectors may also include nucleic acid sequences that serve other functions.

As used in the present invention, the term "host cell" includes a eukaryote and a prokaryote, and refers to any transformable organism capable of replicating the vector or expressing a gene encoded by the vector. The host cell may be transfected or transformed by the vector, which means a process in which an exogenous nucleic acid molecule is delivered or introduced into the host cell.

In an embodiment, the host cell may be bacteria or animal cell, the animal cell line may be a CHO cell, a HEK cell or a NSO cell, and the bacteria may be Escherichia coli.

In an aspect, the present invention relates to an antibody therapeutic agent including the Fc domain variant of the present invention.

In an embodiment, the Fc domain variant according to the present invention or a protein conjugate including the Fc domain variant may be bound and used with various bioactive polypeptides, such as cytokine, interleukin, interleukin binding protein, enzyme, antibody, growth factor, transcriptional regulator, blood factor, vaccine, structural protein, ligand protein or receptor, cell surface antigen, and receptor antagonist, used for the purpose of treating or preventing human diseases, and derivatives and analogues thereof.

In an embodiment, an antibody drug may be bound to the Fc domain variant according to the present invention or the protein conjugate including the Fc domain variant, and the antibody drug for cancer therapy may be trastzumab, cetuximab, bevacizumab, rituximab, basiliximab, infliximab, ipilimumab, pembrolizumab, nivolumab, atezolizumab, or avelumab.

In the antibody therapeutic agent, a mechanism for collecting and delivering immune cells to a target antigen is one of the most important mechanisms, and since the Fc domain of the antibody plays a critical role in the collection of immune cells and antibody-dependent cell-mediated cytotoxicity (ADCC), the Fc variant with increased selective binding affinity to the Fc gamma receptor of the present invention is advantageous to be used as an antibody for treatment. In particular, since the ADCC function of the antibody depends on interaction with the Fc gamma receptor (FcyR) present on the surface of many cells, and a type of immune cell to be collected is determined depending on which Fc receptor among five Fc receptors in humans the antibody binds to, attempts to modify the antibody so as to collect a specific cell are very important in the therapeutic field.

In an aspect, the present invention relates to a pharmaceutical composition for preventing or treating cancer including an Fc domain variant of the present invention, an antibody including the Fc domain variant, or an immunologically active fragment thereof as an active ingredient.

In an embodiment, the cancer may be any one selected from the group consisting of brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma.

In an embodiment, the antibody including the Fc domain variant of the present invention, or the immunologically active fragment thereof has high FcyRIIIa binding selectivity to have a high A/I ratio, thereby increasing an effector function through natural killer cells (NK cells) and increasing antibody dependent cellular cytotoxicity (ADCC). Since the natural killer cells (NK cells), which have the strongest cancer cell killing effect, unlike other immune cells (e.g., monocytes, macrophages, dendritic cells), express FcyRIIIa on the surface, but do not express FcyRI, FcyRIIa, FcyRIIb, and FcyRIIIb, an antibody including the Fc domain variant with FcyRIIIa binding selectivity of the present invention or an immunologically active fragment thereof may maximize a cancer cell death mechanism through NK cells.

In an embodiment, the composition of the present invention may further include an immunogenic cell death inducing agent. The immunogenic cell death inducing agent may be any one or more selected from the group consisting of an anthracycline-based anticancer agent, a taxane-based anticancer agent, an anti-EGFR antibody, a BK channel agonist, bortezomib, cardiac glycoside, a cyclophosmide anticancer agent, a GADD34/PP1 inhibitor, LV-tSMAC, Measles virus, bleomycin, mitoxantrone, or oxaliplatin. The anthracycline-based anticancer agent may include daunorubicin, doxorubicin, epirubicin, idarubicin, pixantrone, sabarubicin, or valrubicin, and the taxane-based anticancer agent may be paclitaxel or docetaxel.

The pharmaceutical composition for preventing or treating cancer of the present invention is administered together with a chemical anticancer drug (anticancer agent) and the like to increase a cancer therapy effect of conventional anticancer agents through the death effect of cancer cells. Combined administration may be performed simultaneously or sequentially with the anticancer agent. Examples of the anticancer agent include DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anti-cancer antibiotics such as dactinomycin (actinomycin D), plicamycin, and mitomycin C; plant alkaloids such as vincristine, vinblastine, etoposide, teniposide, topotecan, and iridotecan, and the like, but are not limited thereto.

As used herein, the term "prevention" means all actions that inhibit or delay the occurrence, spread, and recurrence of cancer by administration of the pharmaceutical composition according to the present invention.

As used herein, the term "treatment" refers to all actions that improve or beneficially change the death of cancer cells or the symptoms of cancer by administration of the composition of the present invention. Those skilled in the art to which the present invention pertains will be able to determine the degree of improvement, enhancement and treatment by knowing the exact criteria of a disease for which the composition of the present invention is effective by referring to data presented by the Korean Academy of Medical Sciences, etc.

As used herein, the term "therapeutically effective amount" used in conjunction with the active ingredients refer to the amount of pharmaceutically acceptable salt of the composition effective for preventing or treating a target disease, and the therapeutically effective amount of the composition of the present invention may vary depending on many factors, such as a method of administration, a target site, the condition of a patient, and the like. Accordingly, when used in the human body, the dose should be determined as an appropriate amount in consideration of both safety and efficiency. It is also possible to estimate the amount used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective amount. As used herein, the term "pharmaceutically effective amount" refers to an amount enough to treat the disease at a reasonable benefit/risk ratio applicable to medical treatment and not to cause side effects. The effective dose level may be determined according to factors including the health condition of a patient, the type and severity of cancer, the activity of a drug, the sensitivity to a drug, a method of administration, a time of administration, a route of administration, an excretion rate, duration of treatment, and simultaneously used drugs, and other factors well-known in the medical field. The composition of the present invention may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present invention may further include a pharmaceutically acceptable additive. At this time, the pharmaceutically acceptable additive may use starch, gelatinized starch, microcrystalline cellulose, lactose, povidone, colloidal silicon dioxide, calcium hydrogen phosphate, lactose, mannitol, syrup, arabic gum, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present invention is preferably included in an amount of 0.1 part by weight to 90 parts by weight based on the composition, but is not limited thereto.

The composition of the present invention may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using as a suitable method in the art or a method disclosed in Remington's Pharmaceutical Science (Mack Publishing Company, Easton PA, 18th, 1990).

The composition of the present invention may be administered parenterally (e.g., applied with injectable formulations intravenously, subcutaneously, intraperitoneally or topically) or orally according to a desired method, and the range of the dose may vary depending on the body weight, age, sex, and health condition of a patient, a diet, an administration time, an administration method, an excretion rate, the severity of a disease, and the like. A daily dose of the composition according to the present invention is 0.0001 to 10 mg/ml, preferably 0.0001 to 5 mg/ml, and more preferably administered once to several times a day.

Liquid formulations for oral administration of the composition of the present invention correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

The present invention includes a method for preparing a sustained drug formulation by covalently linking the Fc domain variant to a bioactive polypeptide through a non-peptidyl polymer.

The preparation method according to the present invention may include covalently linking a bioactive polypeptide and an Fc domain variant through a non-peptide polymer having a reactive group at a terminal; and separating a conjugate in which the bioactive polypeptide, the non-peptide polymer, and the Fc domain variant are covalently linked.

In an aspect, the present invention relates to a method for preparing a human antibody Fc domain variant with improved selective binding affinity to FcyRIIIa, including a) culturing a host cell including a vector containing a nucleic acid molecule encoding an Fc domain variant of the present invention; and b) recovering a polypeptide expressed by the host cell.

In an aspect, the present invention relates to a method for preparing a glycosylated antibody with improved selective binding affinity to FcyRIIIa, including a) culturing a host cell including a vector containing a nucleic acid molecule encoding an antibody of the present invention or an immunologically active fragment thereof; and b) purifying the antibody expressed from the host cell.

In an embodiment, the purification of the antibody may include filtration, HPLC, anion exchange or cation exchange, high performance liquid chromatography (HPLC), affinity chromatography, or a combination thereof, preferably affinity chromatography using Protein A.

In an aspect, the present invention relates to a use of the antibody of the present invention or an immunologically active fragment thereof to be used for preparation of an antibody therapeutic agent.

In an aspect, the present invention relates to a use of the antibody of the present invention or an immunologically active fragment thereof for preventing or treating cancer.

In an aspect, the present invention relates to a method for treating cancer including administering the antibody of the present invention or an immunologically active fragment thereof in a pharmaceutically effective amount to a subject suffering from cancer.

### [Modes of the Invention]

Hereinafter, the present invention will be described in more detail with reference to the following Examples. However, the following Examples are only intended to embody the contents of the present invention, and the present invention is not limited thereto.

### Example 1. Preparation and analysis of trastuzumab Fc variants consisting of simple combinations of Fc mutations

### 1-1. Preparation of simple combined trastuzumab Fc variants

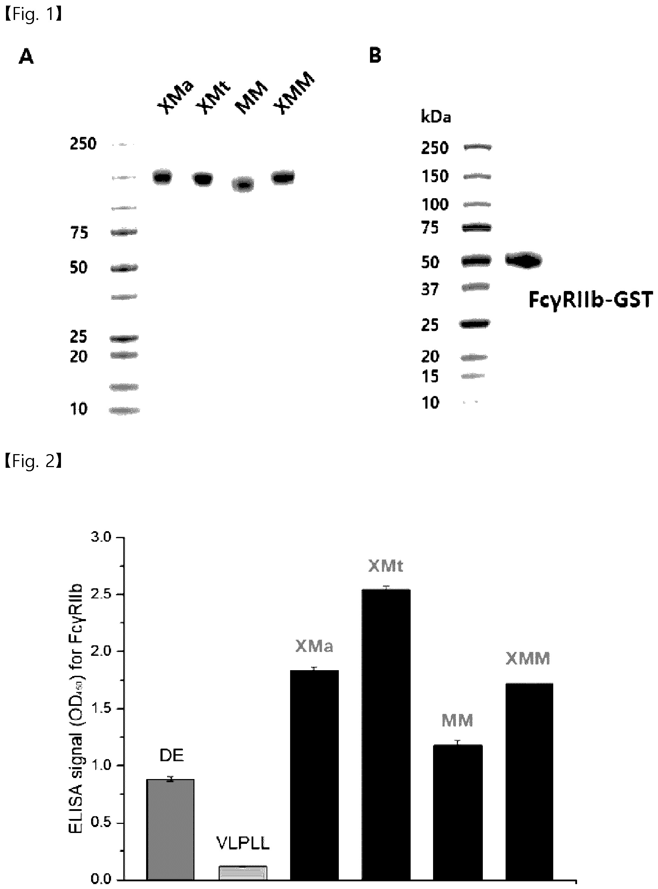

In order to confirm the effect of simple combinations of previously known trastuzumab Fc variants, Fc variants consisting of simple combinations of DEL variants (S239D/I332E/A330L: 9-fold improved A/I ratio compared to wild-type Fc), LPLIL variants (F243L/R292P/Y300L/V305I/P396L: 10-fold improved binding affinity to FcyRIIIa-158V), and IQ variants (P247 II A339Q: 6-fold improved ability to induce ADCC) were prepared (XMa: S239D/F243L/R292P/Y300L/V305I/A330L/I332E/P396L, XMt: S239D/P247I/A330L/I332E/A339Q, MM: F243L/P247I/R292P/Y300L/V305I/A339Q/P396L and XMM: S239D/F243L/P247I/R292P/Y300L/V305I/A330L/I332E/A339Q/P396L) (Table 1). Temporary expression was induced by cloning Fc variants into a heavy chain gene of trastuzumab, a model antibody, and co-transfecting Expi293F cells with PEI (polyethylenimine) (Polyscience, 23966) along with a light chain gene. The co-transfected Expi293F cells were cultured for 7 days under conditions of 37°C, 125 rpm, and 8% CO₂, the culture solution was recovered and equilibrated with PBS, and then trastuzumab Fc variants were purified through Protein A affinity chromatography (FIG. 1A).

### 1-2. Production of FcyRIIb-GST

FcyRIIb-GST was produced to confirm the binding affinity of simple combined trastuzumab Fc variants to FcyRIIb, an Fc receptor that down-regulated immune responses to attenuate ADCC activity. Specifically, a plasmid in which a gene in which GST was fused to the C-terminus of FcyRIIb was cloned into an animal cell expression vector was transfected into the Expi293F cells using PEI and temporarily expressed for 7 days under conditions of 37°C, 125 rpm, and 8% CO₂. Then, the cell culture solution was recovered, equilibrated with PBS, and high-purity FcyRIIb-GST was secured through anti-GST affinity chromatography (FIG. 1B).

### 1-3. Analysis of binding affinity to FcyRIIb of simple combined trastuzumab Fc variants

ELISA assay was performed to confirm the binding affinity to FcyRIIb of trastuzumab Fc variants(XMa, XMt, MM, and XMM) purified and prepared in Example 1-1. Specifically, 50 µl of FcyRIIb-GST diluted to 4 µg/ml in 0.05 M Na₂CO₃ pH 9.6 was immobilized on a flat bottom polystyrene high bind 96-well microplate (Costar, 3590) for 16 hours at 4°C, and then blocked with 100 µl of 4% skim milk (GenomicBase, SKI400) at room temperature for 2 hours. Thereafter, 50 µl of the glycosylated trastuzumab Fc variants prepared in Example 1-1 washed four times with 180 µl of 0.05% PBST and diluted with 1% skim milk were dispensed into each well, and reacted at room temperature for 1 hour. The wells were washed, and added with 50 µl of HRP-Protein L (GenScript, M00098), and then antibody reaction was performed for 1 hour at room temperature, and then washed. Thereafter, 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek). As a result, it was confirmed that trastuzumab Fc variants consisting of simple combinations of known mutations had significantly higher binding affinity to FcyRIIb than ADCC-improved glycosylated Fc variants approved by the US FDA as therapeutic agents for B-cell lymphoma and Her2-positive breast cancer, respectively (DE (S239E/I332E) approved by the US FDA on July 31, 2020 as a therapeutic agent for B-cell lymphoma (Monjuvi, Tafasitamab) and VLPLL (L235V/F243L/R292P/Y300L/P396L) approved by the US FDA on December 16, 2020 as a Her2-positive breast cancer therapeutic agent (Margenza, Margetuximab)) (FIG. 2) to have a negative effect on inducing ADCC activity.

### Example 2. Construction of mammalian cell display system for screening glycosylated Fc variants

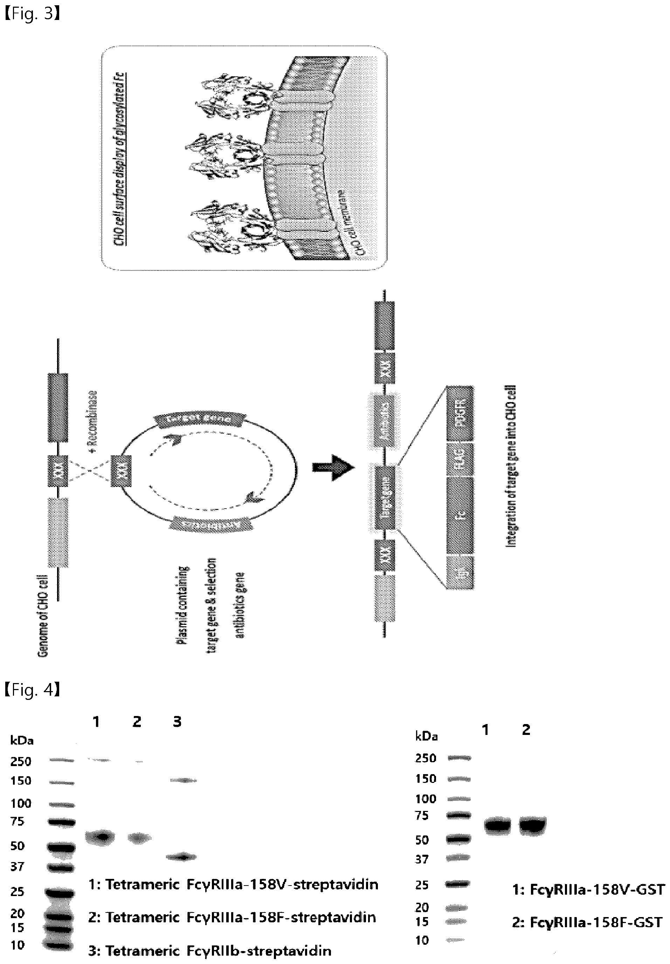

An Fc library was constructed to find optimal combinations of Fc mutations to maximize ability to induce ADCC and to discover new glycosylated Fc variants. Specifically, to display and stably screen glycosylated Fc on the surface of a mammalian cell, a FLP-FRT gene recombination system, which was used to construct stable cell lines, was used, and a platelet-derived growth factor receptor (PDGFR) transmembrane domain was fused to the C-terminus of Fc to be displayed on the cell membrane. A Fc-PDGFR gene was cloned and prepared by insertion into pcDNA5/FRT plasmid (Invitrogen, V601020), which contains a FRT site. The corresponding plasmid was co-transfected into CHO cells (Invitrogen, R75807) into which the FRT site was inserted, together with a pOG44 plasmid (Invitrogen, V600520) expressing FLP, an enzyme that causes genetic recombination, to induce gene recombination, and thus a CHO cell line stably expressing glycosylated Fc was constructed by integrating Fc-PDGFR DNA into chromosomal DNA of CHO cells (FIG. 3). At this time, in order to screen only CHO cells in which gene integration occurred after transfection, a hygromycin-B resistance gene was integrated together, and a mammalian cell display system for screening glycosylated Fc variants was constructed by treating 500 µg/ml of hygromycin-B (Invitrogen, 10687010) to select a CHO cell line stably expressing glycosylated Fc.

### Example 3. Preparation of FcyRs for binding affinity analysis

### 3-1. Expression and purification

To conduct FACS screening of the established CHO cell display system and Fc library stable expression cell lines of glycosylated Fc and analyze FcyRs binding affinity of Fc variants to be discovered, FcyRIIIa and FcyRIIb were prepared and produced. Since FcyRIIIa and FcyRIIb among FcyRs were known as a low affinity receptor with low binding affinity to Fc, streptavidin was fused to the C-terminus of each receptor to prepare tetrameric FcyRIIIa-158V-streptavidin-His, tetrameric FcyRIIIa-158F-streptavidin-His, and tetrameric FcyRIIb-streptavidin-His with improved visible binding affinity to Fc and efficient FACS screening. In addition, to analyze the FcyRs binding affinity of the glycosylated Fc variants to be discovered by ELISA, FcγRIIIa-158V-GST and FcyRIIIa-158F-GST were prepared. Respective proteins were cloned and prepared into an animal cell expression vector, transfected into Expi293F cells using PEI, and cultured for 7 days under conditions of 37°C, 125 rpm, and 8% CO₂. After culturing, the supernatant was recovered, equilibrated with PBS, and purified by Ni-NTA (Anti-His) or anti-GST affinity chromatography. As a result, high-purity tetrameric FcyRIIIa-158V-streptavidin, tetrameric FcyRIIIa-158F-streptavidin, tetrameric FcyRIIb-streptavidin, FcγRIIIa-158V-GST and FcyRIIIa-158F-GST were purified (FIG. 4).

### 3-2. Functional verification

Among the proteins prepared and produced in Example 3-1, the tetrameric FcyRIIIa-streptavidin prepared for FACS screening was labeled with fluorescent dyes of Alexa488 (Invitrogen, A10235) and Alexa647 (Invitrogen, A20173), and then prepared to enable sorting of Fc variants through competitive binding with non-fluorescent tetrameric FcyRIIb-streptavidin. In order to check the expression and levels of the displayed Fc variants, FITC (Invitrogen, F6434) was conjugated and prepared to Protein A (Amicogen, 1070020), in which the binding site did not overlap with FcyRs. Conjugation of fluorescent dyes (alexa488, Alexa647, and FITC) was performed according to the manual provided by each manufacturer. Fluorescent-labeled tetrameric FcyRIIIa-streptavidin-Alexa488, tetrameric FcyRIIIa-streptavidin-Alexa647, and Protein A-FITC were induced to bind to wild-type Fc and an Fc variant (Fc-T299L) from which FcyRs binding affinity was removed, which were displayed on CHO cells, respectively, and then the activity was checked.

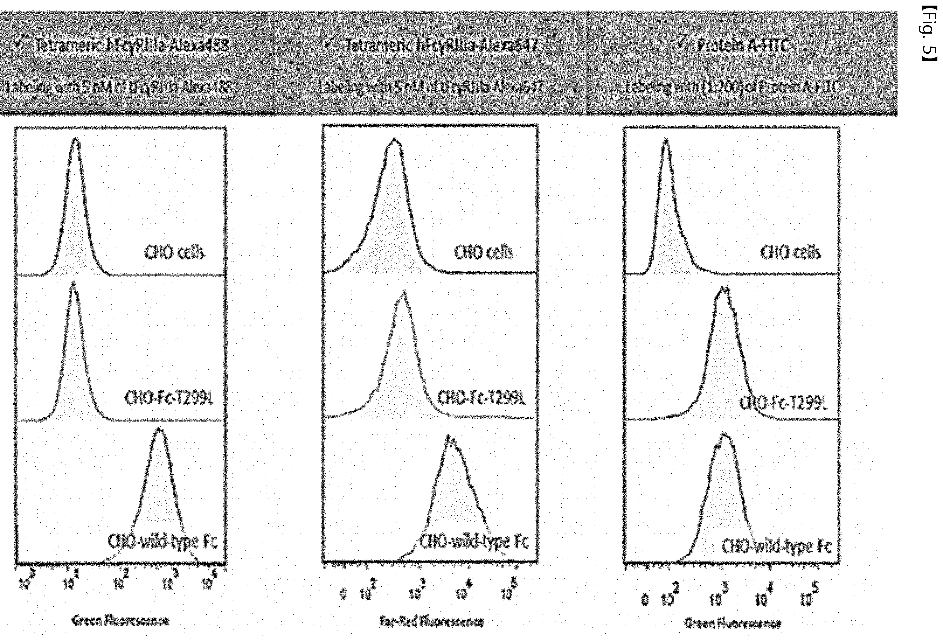

As a result, Protein A-FITC had normal activity through a fluorescence signal caused by Protein A-FITC binding, and the wild-type Fc and Fc-T299L displayed on the CHO cells were stably expressed to have similar expression levels to each other (FIG. 5). In addition, tetrameric FcyRIIIa-streptavidin-Alexa488 and tetrameric FcyRIIIa-streptavidin-Alexa647 also bound normally to wild-type Fc, but did not bind to Fc-T299L, the Fc variant from which FcyRs binding affinity has been removed (FIG. 5). Alexa488/647 fluorescent-labeled FcyRIIIa also had excellent activity, and it was successfully verified that the Fcs displayed on the CHO cells were expressed normally and performed respective functions.

### Example 4. Construction of glycosylated Fc variant library with improved selective binding affinity to FcyRIIIa using mammalian cell display

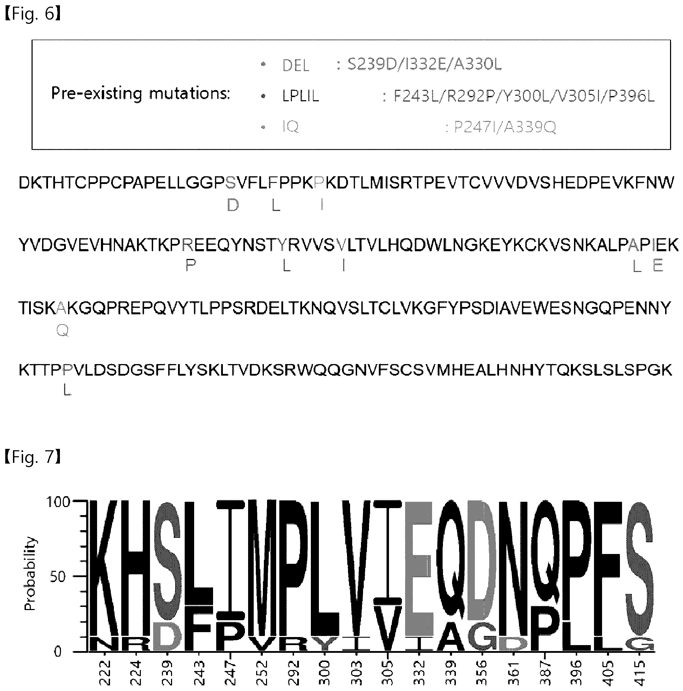

Glycosylated Fc was engineered using the established CHO cell Fc display system, and a library was constructed to select glycosylated Fc variants with improved binding affinity to FcyRIIIa. Using the library, the mutations of the DEL variant, the LPLIL variant, and the IQ variant were shuffled to search for an optimal mutation combination capable of maximizing FcyRIIIa binding (FIG. 6). The library genes were co-transfected with an FLP expression plasmid in the same manner as the method for preparing the Fc stable expression mammalian cell line established in Example 2 above, and then subjected to a screening process using the hygromycin-B medium to produce a glycosylated Fc variant library stable expression CHO cell line.

### Example 5. Screening of Fc variants with improved selective binding affinity to FcyRIIIa using mammalian cell display

In order to select glycosylated Fc with improved binding affinity to FcyRIIIa using the CHO cell Fc display system established in Example 2 and the Fc library stable expression CHO cell line established in Example 4, in the library stable expression CHO cell line, the binding of FcyRIIIa-Alexa647, Protein A-FITC or FcyRIIIa-Alexa647 to non-fluorescent FcyRIIb and Protein A-FITC was induced, and then the expression level by Protein A-FITC and FcyRIIIa binding were simultaneously monitored to perform FACS screening of 1R. As a result, a cell population expected to show excellent binding affinity to FcyRIIIa and selectivity was selected, CHO cells expressing selected glycosylated Fc variants were recovered through genomic DNA prep., and then base sequences were confirmed to select finally engineered glycosylated Fc variants (FIG. 7).

### Example 6. Analysis of binding affinity to FcyRIIIa of trastuzumab Fc variants

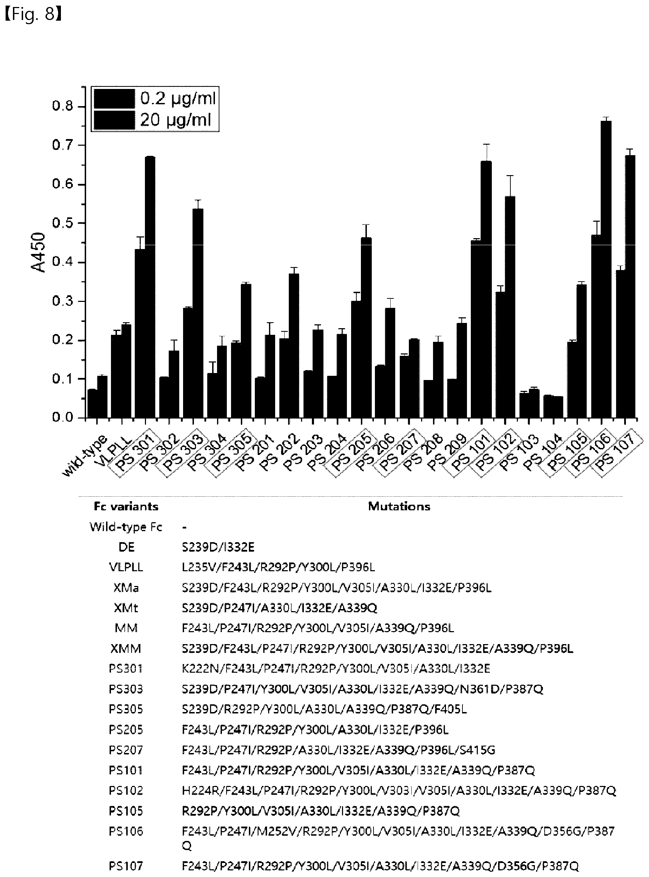

In order to express and purify glycosylated Fc variants, an expression vector was prepared by cloning the Fc variants into a trastuzumab heavy chain gene as a model antibody. Specifically, a heavy chain gene and a light chain gene of the variants were first mixed in 3 ml of a Freestyle293 expression culture solution (Gibco, 12338-018) at a ratio of 1 : 1, polyethylenimine (PEI) (Polyscience, 23966) and variant genes were mixed at a ratio of 4 : 1 and incubated at room temperature for 20 minutes, and then mixed with 30 ml of subcultured Expi293F cells at a density of 2 × 10⁶ cells/ml, cultured in a shaking CO₂ incubator under conditions of 37°C, 125 rpm, and 8% CO₂ for 7 days, and then centrifuged to collect only a supernatant. The supernatant was equilibrated with 25×PBS and then filtered through a 0.2 µm syringe filter. 50 µl of the filtered trastuzumab Fc variant expression culture solution was added to a high binding 96-well plate (Costar, 3590) coated with an extracellular domain of Her2 protein overnight at 4°C and immobilized for 1 hour. Thereafter, the culture solution was blocked with 100 µl of 4% skim milk (GenomicBase, SKI400) at room temperature for 2 hours. After washed four times with 180 µl of 0.05% PBST, 50 µl each of 20 µg/ml and 0.02 µg/ml FcyRIIIa-GST in 1% skim milk was added to each well and reacted at room temperature for 1 hour. After washing, 50 µl of goat anti-GST-HRP (GE Healthcare, RPN1236V) was treated to each well, antibody reaction was performed for 1 hour at room temperature, and then each well was washed. 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek). As a result, it was confirmed that among the novel Fc variants developed in the present invention, PS301, PS303, PS305, PS205, PS207, PS101, PS102, PS105, PS106 and PS107 (Table 1) had similar or significantly high binding affinity to FcyRIIIa to a VLPLL variant, the previously known margetuximab, approved as a therapeutic agent for Her2-positive breast cancer (FIG. 8).

**[Table 1]**

| # | Mutations | SEQ ID NOs: |
|---|---|---|
| PS301 | K222N/F243L/P247I/R292P/Y300L/V305I/A330L/I332E | 22 and 37 |
| PS303 | S239D/P247I/Y300L/V305I/A330L/I332E/A339Q/N361D/ P387Q | 23 and 38 |
| PS305 | S239D/R292P/Y300L/A330L/A339Q/P387Q/F405L | 24 and 39 |
| PS205 | F243L/P247I/R292P/Y300L/A330L/I332E/P396L | 25 and 40 |
| PS207 | F243L/P247I/R292P/A330L/I332E/A339Q/P396L/S415G | 26 and 41 |
| PS101 | F243L/P247I/R292P/Y300L/V305I/A330L/I332E/A339Q/ P387Q | 27 and 42 |
| PS102 | H224R/F243L/P247I/R292P/Y300L/V303I/V305I/A330L/I 332E/A339Q/P387Q | 28 and 43 |
| PS105 | R292P/Y300L/V305I/A330L/I332E/A339Q/P387Q | 29 and 44 |
| PS106 | F243L/P247I/M252V/R292P/Y300L/V305I/A330L/I332E/ A339Q/D356G/P387Q | 30 and 45 |
| PS107 | F243L/P247I/R292P/Y300L/V305I/A330L/I332E/A339Q/ D356G/P387Q | 31 and 46 |
| XMa | S239D/F243L/R292P/Y300L/V305I/A330L/I332E/P396L | 32 and 47 |
| XMt | S239D/P247I/A330L/I332E/A339Q | 33 and 48 |
| MM | F243L/P247I/R292P/Y300L/V305I/A339Q/P396L | 34 and 49 |
| XMM | S239D/F243L/P247I/R292P/Y300L/V305I/A330L/I332E/ A339Q/P396L | 35 and 50 |

### Example 7. Purification of glycosylated trastuzumab Fc variants with improved binding affinity to FcγRIIIa

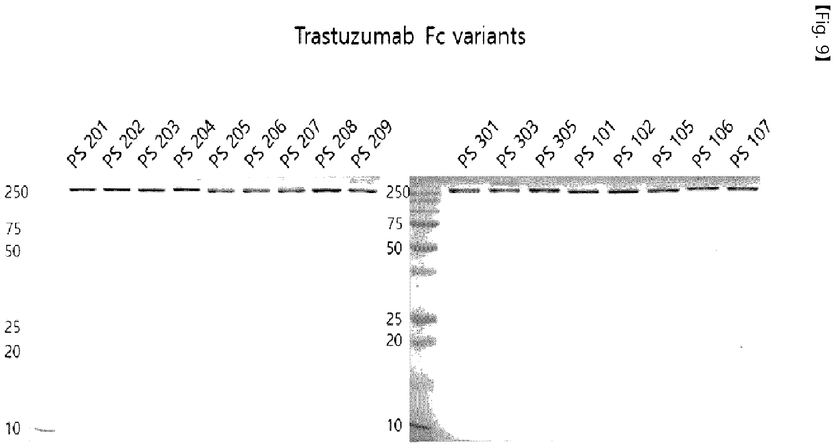

In order to purify trastuzumab Fc variants confirmed to have high binding affinity to FcyRIIIa through culture medium ELISA in Example 6, the variants were added with a Protein A resin, stirred at 4°C for 16 hours, and then spun down to recover the resin, and then washed with 2 ml PBS, eluted with 300 µl of a 100 mM glycine pH 2.7 buffer, and then neutralized using 100 µl of 1 M Tris-HCl pH 8.0. To change the buffer, Amicon Ultra-4 centrifugal filter units 30K (Merck Millipore, UFC503096) were used. As a result, it was confirmed that high-purity glycosylated antibody trastuzumab Fc variants were purified (FIG. 9).

### Example 8. ELISA assay of binding affinity to FcγRIIIa and FcγRIIb of glycosylated trastuzumab Fc variants

ELISA assay was performed to confirm the binding affinity to FcyRIIIa and FcyRIIb of the glycosylated trastuzumab Fc variants expressed and purified in Example 7. Specifically, 50 µl of FcyRs-GSTs (FcyRIIIa-158V-GST, FcyRIIIa-158F-GST and FcyRIIb-GST) diluted to 4 µg/ml in 0.05 M Na₂CO₃ (pH 9.6) were dispensed on a flat bottom polystyrene high bind 96-well plate (Costar, 3590), respectively, immobilized for 16 hours at 4°C, and then blocked with 100 µl of 4% skim milk (GenomicBase, SKI400) at room temperature for 2 hours. Thereafter, 50 µl of the glycosylated trastuzumab Fc variants washed four times with 180 µl of 0.05% PBST and serially diluted with 1% skim milk were dispensed into each well, and reacted at room temperature for 1 hour. The wells were washed, and treated with 50 µl of HRP-Protein L (GenScript, M00098), and then antibody reaction was performed for 1 hour at room temperature, and then washed. Thereafter, 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek).

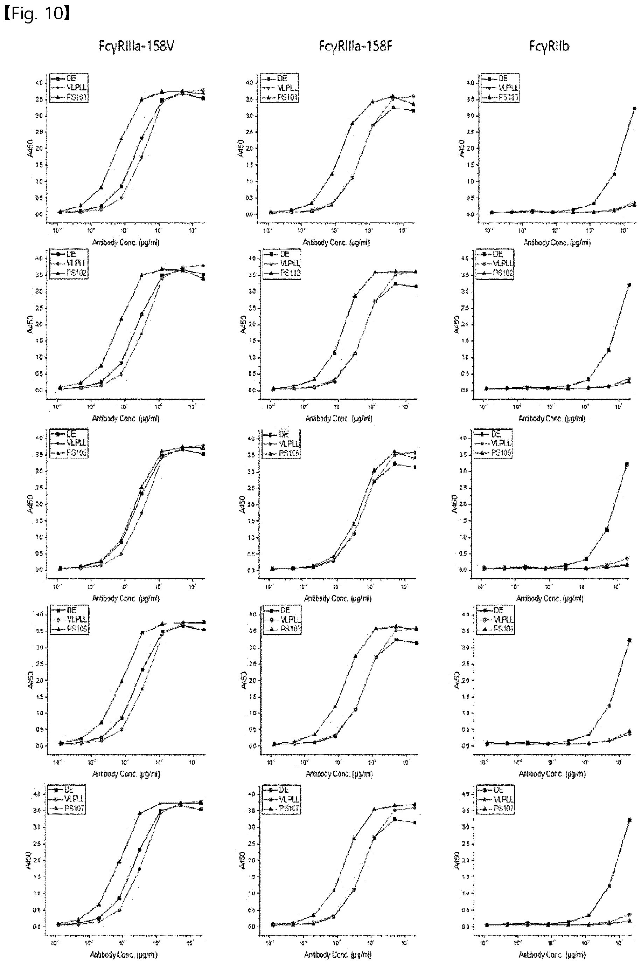

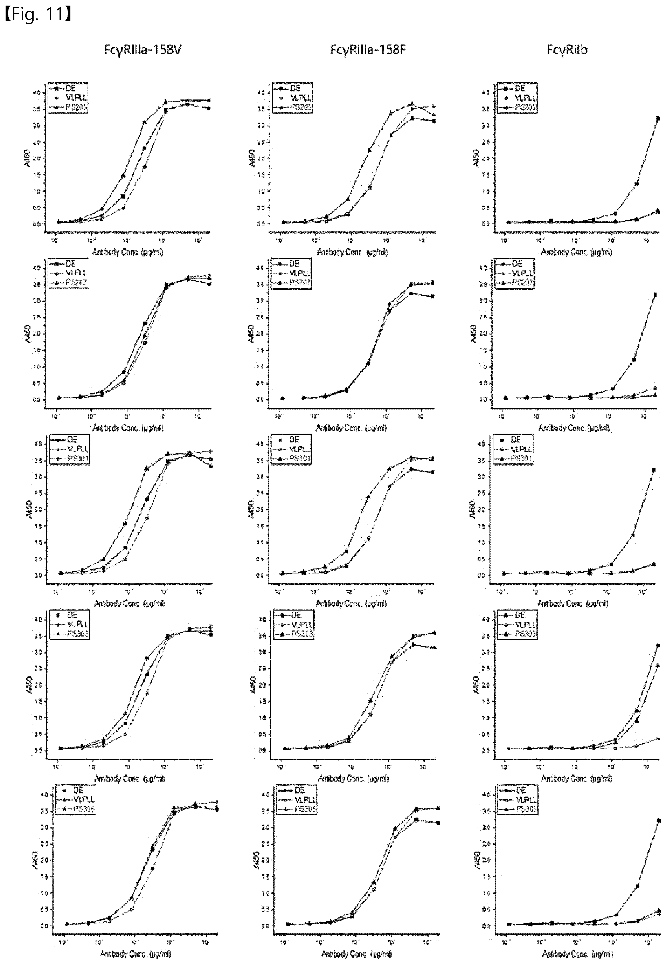

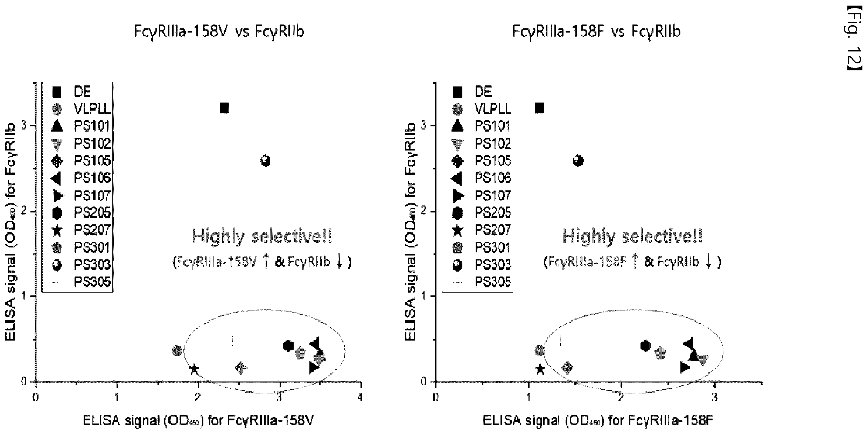

As a result, the glycosylated trastuzumab Fc variants of the present invention had significantly higher FcyRIIIa/ FcyRIIb selective binding affinity than glycosylated Fc variants (DE and VLPLL) approved by the US FDA as therapeutic agents for B-cell lymphoma and Her2-positive breast cancer, respectively, and Fc variants XMa, XMt, MM and XMM consisting of simple combinations of previously known mutations (FIGS. 10 and 11). In particular, some mutations were added and deleted selectively by searching a large library of glycosylated antibody Fc variants rather than simple combinations of known function-improved Fc variant mutations, and Fc variants containing optimized mutations were very effective in inducing FcγRIIIa/ FcyRIIb selective binding affinity (FIG. 12), and divided into three groups as shown in Table 2 below according to their bonding properties.

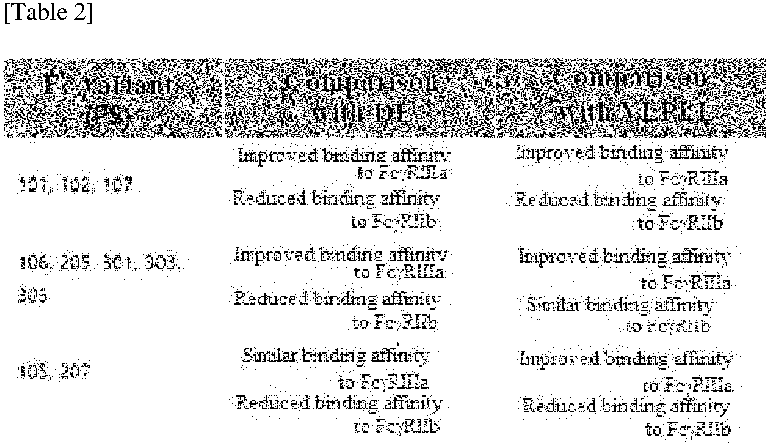

### Example 9. Measurement of FcγRIIIa and FcγRIIb binding constants of glycosylated trastuzumab Fc variants

Among the glycosylated trastuzumab Fc variants expressed and purified in Example 7, the quantitative binding constants of PS101, PS102, and PS107 variants were measured using Octet R8 (Sartorius) equipment. Specifically, a baseline was set with PBS for 60 seconds in a Ni-NTA biosensor hydrated for 10 minutes, and then 5 µg/ml of FcyRIIIa-158V-streptavidin-His, FcyRIIIa-15SF-streptavidin-His and FcyRIIb-streptavidin-His were each immobilized. After immobilization, the baseline was set again with PBS, and then trastuzumab Fc variants serially diluted with PBS were associated for 90 seconds. Thereafter, the trastuzumab Fc variants were dissociated with PBS for 150 seconds to obtain a full kinetics sensorgram, and kinetic parameters including quantitative binding constants were obtained using Octet BLI Discovery 12.2 software.

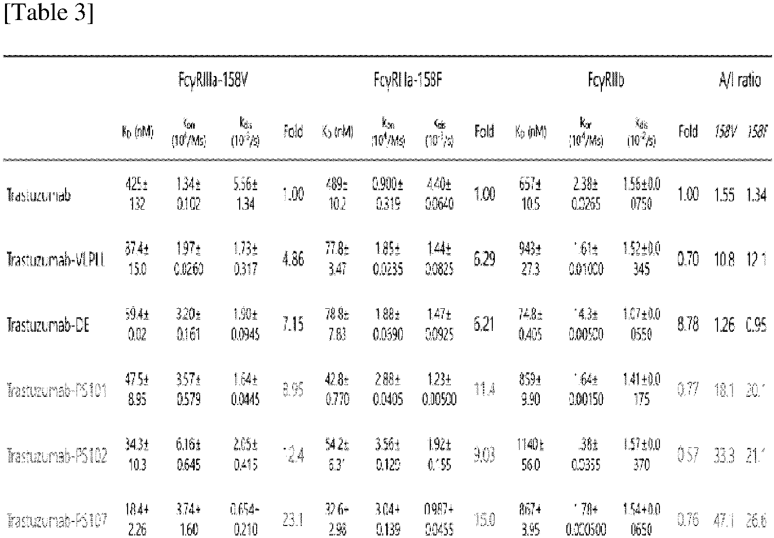

As a result, it was confirmed that three types of trastuzumab Fc variants (trastuzumab-PS101, trastuzumab-PS102 and trastuzumab-PS107) discovered had maximum 23-fold improved binding affinity to FcyRIIIa-158V and maximum 15-fold improved binding affinity to FcyRIIIa-158F and 1.8-fold lowered binding affinity to FcyRIIb compared to wild-type trastuzumab (trastuzumab-PS101 was FcyRIIIa-158V: 8.95-fold, FcyRIIIa-158F: 11.4-fold, and FcyRIIb: 0.77-fold; trastuzumab-PS102 was FcyRIIIa-158V: 12.4-fold, FcyRIIIa-158F: 9.03-fold and FcyRIIb: 0.57-fold; and trastuzumab-PS107 was FcyRIIIa-158V: 23.1-fold, FcγRIIIa-158F: 15.0-fold, and FcyRIIb: 0.76-fold) (Table 3).

### Example 10. Analysis of binding to HER2 expression target cell of glycosylated Trastuzumab Fc variants

The maintenance of antigen binding affinity was analyzed using SKBR-3, a HER2-expressing cancer cell line, which was an antigen of trastuzumab, after introducing the Fc variants of the present invention into a model antibody (trastuzumab). Specifically, 10 nM of the trastuzumab Fc variants (TRZ-PS101, TRZ-PS102 and TRZ-PS107) of the present invention and Protein A-FITC were added to 4 × 10⁶ SKBR-3, incubated in ice for 30 minutes, and then washed with PBS twice to prepare samples according to each Fc variant. The prepared samples were analyzed using a BD FACSLyric flow cytometer to confirm the degree and presence of binding according to the FITC fluorescence intensity.

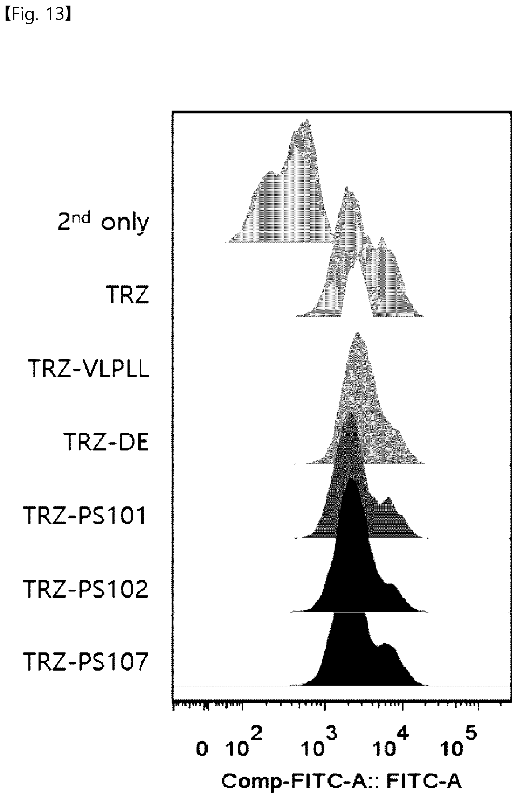

As a result, it was confirmed that the antigen binding affinities of wild-type trastuzumab without introduced Fc mutations and trastuzumab Fc variants introduced with the Fc variants of the present invention were the same (FIG. 13). Through this, it may be seen that the introducing of the Fc variant of the present invention does not affect antigen binding.

### Example 11. Analysis of binding affinity to cell membrane expression FcγRIIIa of glycosylated trastuzumab Fc variants

To mimic the binding of FcyRIIIa expressed on the surface of NK cells and glycosylated trastuzumab Fc variants, naive FcyRIIIa was expressed on the surface of CHO cells, and the binding affinity with the trastuzumab Fc variants of the present invention was analyzed. Specifically, using a FLP-FRT gene recombination system, genes of human FcyRIIIa-158V and human FcyRIIIa-158F were cloned into a pcDNA5/FRT plasmid (Invitrogen, V601020) into which a FRT site was inserted, respectively, and prepared. The gene recombination was induced by co-transfecting the corresponding plasmid with a pOG44 plasmid (Invitrogen, V600520) expressing FLP, an enzyme causing genetic recombination, and CHO cells (Invitrogen, R75807) into which the FRT site was inserted, and thus human FcyRIIIa DNA was integrated into the chromosomal DNA of CHO cells to prepare CHO cell lines stably expressing human FcyRIIIa-158V and human FcγRIIIa-158F, respectively. At this time, in order to select only the CHO cells in which gene integration occurred after transfection, a hygromycin-B resistant gene was integrated together, and 500 µg/ml of hygromycin-B (Invitrogen, 10687010) was treated to select CHO cell lines (CHO-FcyRIIIa-158V and CHO-FcγRIIIa-158F) stably expressing human FcyRIIIa. 2.5 nM of glycosylated trastuzumab Fc variants (TRZ-PS101, TRZ-PS102, and TRZ-PS107) and a HER2-Alexa488 fluorescent probe were added together to the prepared CHO-FcyRIIIa-158V and CHO-FcyRIIIa-158F, respectively to induce the binding, and then the fluorescence was analyzed using a BD FACSLyric flow cytometer.

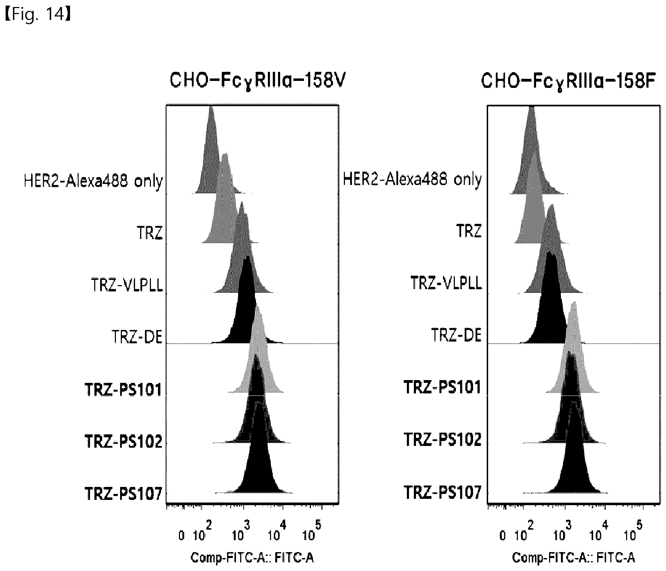

As a result, similarly to the analysis result of the binding affinity to soluble FcyRIIIa, it was shown that the glycosylated trastuzumab Fc variants (TRZ-PS101, TRZ-PS102 and TRZ-PS107) of the present invention had significantly higher binding affinity than positive controls, trastuzumab-DE and trastuzumab-VLPLL (FIG. 14).

### Example 12. Real-time ADCC efficacy analysis of glycosylated trastuzumab Fc variants

In order to confirm the ADCC efficacy of the glycosylated trastuzumab Fc variants of the present invention in real time, cell cytotoxicity was monitored in real time using xCELLigence-RTCA-SP (Agilent) equipment. Specifically, SKBR-3 was coated on an E-plate 96 at 1 × 10⁴ cell/well and then cultured in a 5% CO₂ incubator for 24 hours. After 24 hours, 20 pM of glycosylated trastuzumab Fc variants (TRZ-PS101, TRZ-PS102, and TRZ-PS107) and human PBMC were added and incubated to induce target cell death. At this time, the analysis was performed at an E : T (Effector cell : target cell) ratio of 2 : 1 and 5 : 1, and full lysis was performed by treating 2% Triton X-100, 1% SDS, 100 mM NaCl, and 1 mM EDTA buffer, and %cytolysis was calculated.

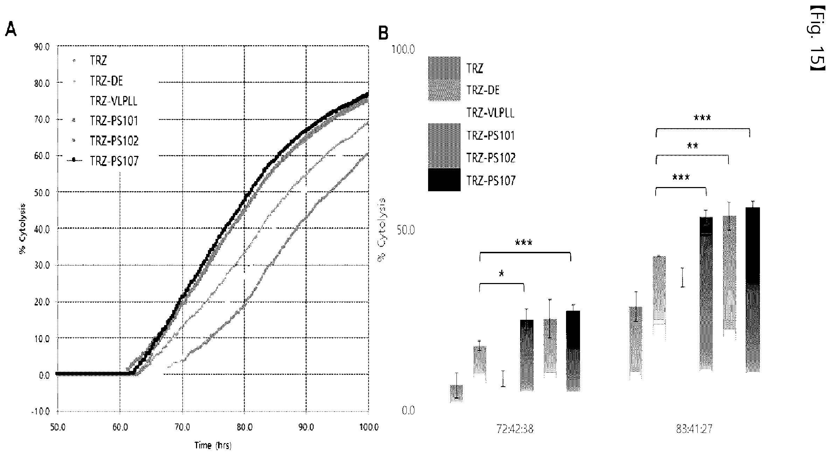

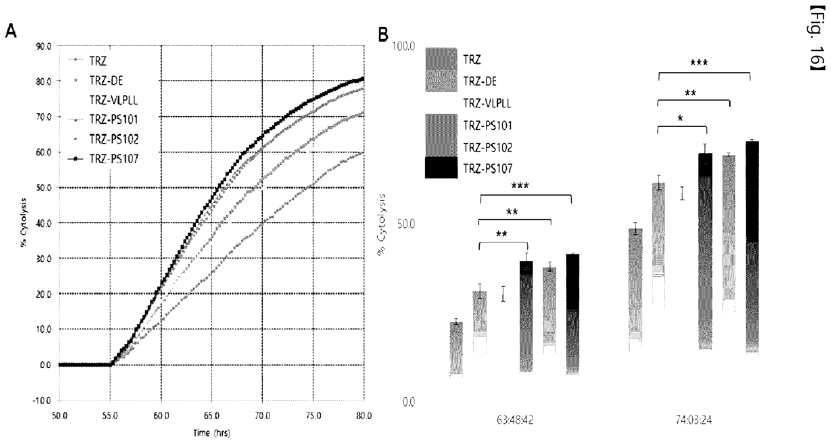

As a result of checking a real-time cell death graph according to each trastuzumab Fc variant, it was shown that at both E : T ratios of 2 : 1 (FIG. 15) and 5 : 1 (FIG. 16), the target cell death effect of the trastuzumab Fc variants of the present invention, trastuzumab-PS101, trastuzumab-PS102 and trastuzumab-PS107 (TRZ-PS101, TRZ-PS102 and TRZ-PS107) was significantly higher than that of a control, wild-type trastuzumab, and positive controls, trastuzumab-DE and trastuzumab-VLPLL. Even in endpoint analysis based on real-time graphs (FIGS. 15A and 16A), it was confirmed that trastuzumab-PS101, trastuzumab-PS102, and trastuzumab-PS107 of the present invention had a significantly excellent % of cytotoxicity (FIGS. 15B and 16B).

### Example 13. Immunogenicity prediction analysis of glycosylated trastuzumab Fc variants

In order to analyze the immunogenicity of the glycosylated trastuzumab Fc variants of the present invention by MHC class II binding, the immunogenicity potential according to their base sequences of the Fc variants of the present invention was confirmed based on IEDB. Specifically, the binding affinity of Fc peptides (PS101, PS102, and PS107) was analyzed by percentile rank with respect to 27 types of HLAs most commonly found in humans (HLA-DRB1*01:01, HLA-DRB1*03:01, HLA-DRB1*04:01, HLA-DRB1*04:05, HLA-DRB1*07:01, HLA-DRB1*08:02, HLA-DRB1*09:01, HLA-DRB1*11:01, HLA-DRB1*12:01, HLA-DRB1*13:02, HLA-DRB1*15:01, HLA-DRB3*01:01, HLA-DRB3*02:02, HLA-DRB4*01:01, HLA-DRB5*01:01, HLA-DQA1*05:01/DQB1*02:01, HLA-DQA1*05:01/DQB1*03:01, HLA-DQA1*03:01/DQB1*03:02, HLA-DQA1*04:01/DQB1*04:02, HLA-DQA1*01:01/DQB1*05:01, HLA-DQA1*01:02/DQB1*06:02, HLA-DPA1*02:01/DPB1*01:01, HLA-DPA1*01:03/DPB 1*02:01, HLA-DPA1*01:03/DPB1*04:01, HLA-DPA1*03:01/DPB1*04:02, HLA-DPA1*02:01/DPB1*05:01, and HLA-DPA1*02:01/DPB1*14:01). At this time, Fc was divided into 15-mer peptide forms and analyzed. Based on a database-based prediction rank, high affinity was indicated in red, intermediate affinity was indicated in orange, and low affinity was indicated in yellow to be shown in a heat map.

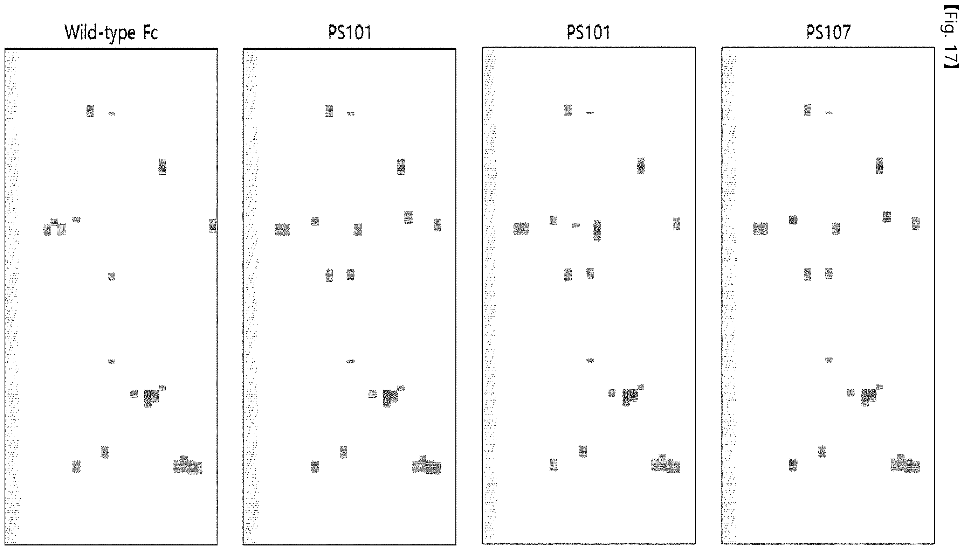

As a result of *in silico* analysis, it was found that there was no significant difference in the immunogenicity prediction result between human IgG1 Fc and the Fc variants of the present invention (FIG. 17).

### Example 14. Analysis of binding affinity to animal Fc receptors of glycosylated trastuzumab Fc variants

### 14-1. Production of mouse FcyRIV and monkey FcyRIII

To verify efficacy in mouse and monkey animal models, expression vectors were prepared by cloning animal Fc receptors corresponding to human FcyRIIIa in GST fusion forms (mouse FcyRIV-GST and cynomolgus monkey FcyRIII-GST). Specifically, a heavy chain gene and a light chain gene of the variants were first mixed in 30 ml of a Freestyle293 expression culture solution (Gibco, 12338-018) at a ratio of 1 : 1, polyethylenimine (PEI) (Polyscience, 23966) and variant genes were mixed at a ratio of 4 : 1 and incubated at room temperature for 20 minutes, and then mixed with 30 ml of subcultured Expi293F cells at a density of 2 × 10⁶ cells/ml, cultured in a shaking CO₂ incubator under conditions of 37°C, 125 rpm, and 8% CO₂ for 7 days, and then centrifuged to collect only the supernatant. The supernatant was equilibrated with 25×PBS, filtered through a 0.2 µm syringe filter, added with a glutathione resin (GenScript, L00206), and stirred at 4°C for 16 hours. After stirring, the sample was placed on a 5 ml disposable polypropylene column (Thermo Fisher Scientific, 29922) to recover the resin, and then washed with 10 CV PBS. After elution with 4 ml of 50 mM Tris-HCl and 10 mM reduced glutathione pH 8.0 buffer, Amicon Ultra-4 centrifugal filter units 3K (Merck Millipore, UFC800324) were used to change the buffer.

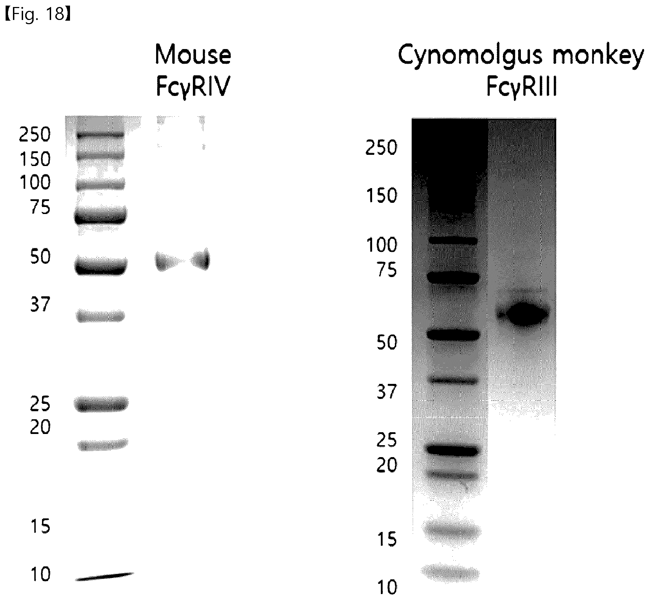

As a result, high-purity mouse FcγRIV-GST and cynomolgus monkey FcγRIII-GST were obtained (FIG. 18).

### 14-2. Analysis of binding affinity to mouse FcγRIV and monkey FcγRIII of glycosylated trastuzumab Fc variants

To confirm the binding affinity to animal Fc receptors corresponding to human FcγRIIIa (mouse FcγRIV and monkey FcγRIII) of trastuzumab Fc variants of the present invention (TRZ-PS101, TRZ-PS102 and TRZ-PS107), ELISA assay was performed. Specifically, mouse FcγRIV-GST and cynomolgus monkey FcγRIII-GST prepared in Example 14-1 were diluted to 4 µg/ml in 0.05 M Na₂CO₃ pH 9.6, and 50 µl of each was immobilized to a flat bottom polystyrene high bind 96-well microplate (Costar, 3590) at 4°C for 16 hours, and then blocked with 100 µl of 4% skim milk (GenomicBase, SKI400) at room temperature for 2 hours. Thereafter, 50 µl of the glycosylated trastuzumab Fc variants washed four times with 180 µl of 0.05% PBST and serially diluted with 1% skim milk were dispensed into each well, and reacted at room temperature for 1 hour. After washing, antibody reaction was performed for 1 hour at room temperature, and then washed using 50 µl of HRP-Protein L (GenScript, M00098). Thereafter, 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek).

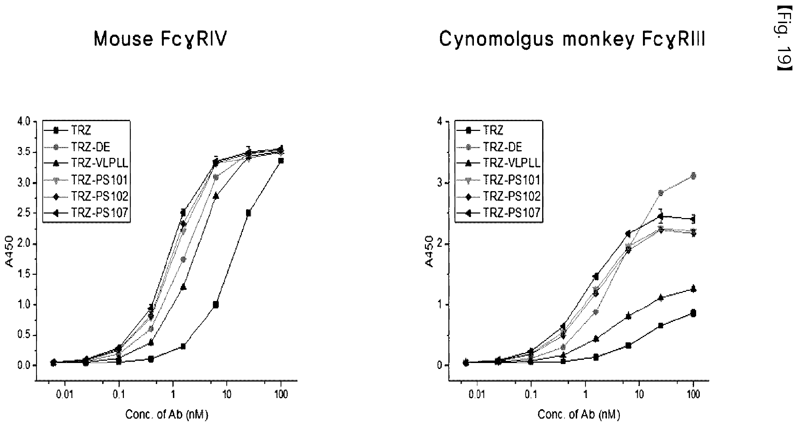

As a result, it was shown that the trastuzumab Fc variants of the present invention (TRZ-PS101, TRZ-PS102 and TRZ-PS107) had significantly higher binding affinity than positive controls, glycosylated Fc variants (DE and VLPLL) even in mouse and cynomolgus monkey Fc receptors similarly to human receptors (FIG. 19).

### Example 15. Preparation of cetuximab or rituximab Fc variants

Expression vectors were prepared by cloning the glycosylated Fc variants (PS101, PS102, and PS107) of the present invention into heavy chain genes of cetuximab and rituximab, which were other model antibodies, respectively. Specifically, a heavy chain gene and a light chain gene of the variants were first mixed in 10 ml of a Freestyle293 expression culture solution (Gibco, 12338-018) at a ratio of 1 : 1, polyethylenimine (PEI) (Polyscience, 23966) and variant genes were mixed at a ratio of 4 : 1 and incubated at room temperature for 20 minutes, and then mixed with 100 ml of subcultured Expi293F cells at a density of 2 × 10⁶ cells/ml, cultured in a shaking CO₂ incubator under conditions of 37°C, 125 rpm, and 8% CO₂ for 7 days, and then centrifuged to collect only the supernatant. The supernatant was equilibrated with 25×PBS, filtered through a 0.2 µm syringe filter, added with a Protein A resin, and stirred at 4°C for 16 hours. After stirring, the sample was placed on a 5 ml disposable polypropylene column (Thermo Fisher Scientific, 29922) to recover the resin, and then washed with 5 ml of PBS. After eluted with 3 ml of 100 mM glycine pH 2.7 buffer, the sample was neutralized using 1 ml of 1 M Tris-HCl pH 8.0. To change the buffer, Amicon Ultra-4 centrifugal filter units 30K (Merck Millipore, UFC503096) were used.

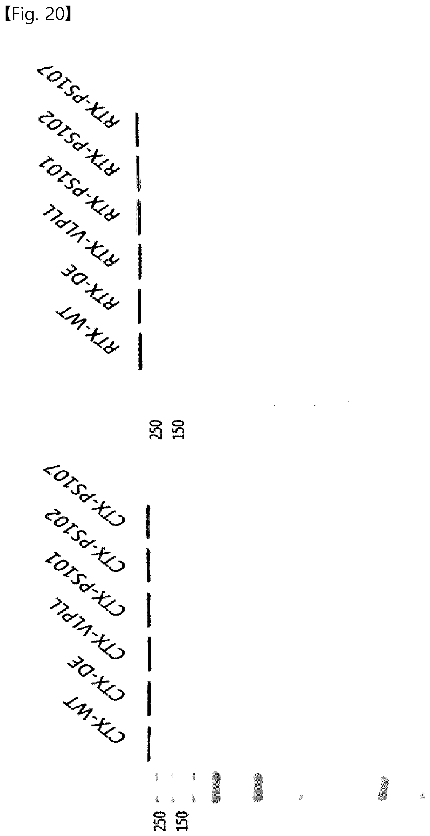

As a result, it was confirmed that high-purity glycosylated antibody cetuximab Fc Variants and rituximab Fc Variants were purified (FIG. 20).

### Example 16. Analysis of binding affinity to Fcγ receptors of glycosylated cetuximab Fc variants

### 16-1. Analysis of binding affinity to human FcγRIIIa and FcγRIIb

ELISA assay was performed to confirm the binding affinity to FcγRIIIa and FcγRIIb of the glycosylated cetuximab Fc variants (CTX-PS101, CTX-PS102, and CTX-PS107) prepared in Example 15. Specifically, 50 µl of FcγRs-GSTs (FcγRIIIa-158V-GST, FcγRIIIa-158F-GST and FcγRIIb-GST) diluted to 4 µg/ml in 0.05 M Na₂CO₃ pH 9.6 were immobilized on a flat bottom polystyrene high bind 96-well plate (Costar, 3590) for 16 hours at 4°C, respectively, and then blocked with 100 µl of 4% skim milk (GenomicBase, SKI400) at room temperature for 2 hours. After washed four times with 180 µl of 0.05% PBST, 50 µl of glycosylated cetuximab Fc variants (CTX-PS101, CTX-PS102, and CTX-PS107) serially diluted with 1% skim milk were dispensed into each well and reacted at room temperature for 1 hour. After washing, antibody reaction was performed for 1 hour at room temperature, and then washed using 50 µl of HRP-Protein L (GenScript, M00098). 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek).

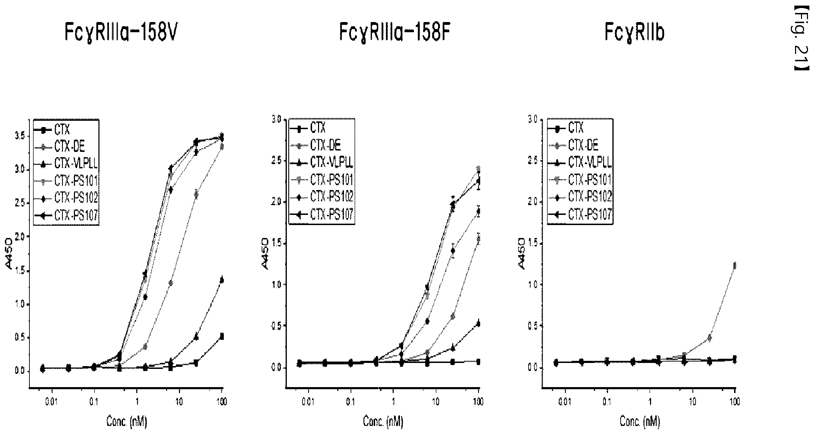

As a result, it was shown that the glycosylated cetuximab Fc variants prepared in the present invention had significantly higher FcγRIIIa/ FcγRIIb selective binding affinity than positive controls, glycosylated Fc variants (DE and VLPLL) (FIG. 21). In particular, it was confirmed that significantly excellent FcγRIIIa/ FcγRIIb selective binding properties could be maintained when Fc variants were introduced into different model therapeutic antibodies.

### 16-2. Analysis of binding affinity to mouse FcγRIV and monkey FcγRIII

ELISA assay was performed to confirm the binding affinity to animal Fc receptors corresponding to human FcγRIIIa of cetuximab Fc variants. Specifically, 50 µl of mouse FcγRIV-GST and cynomolgus monkey FcγRIII-GST diluted to 4 µg/ml in 0.05 M Na₂CO₃ pH 9.6 were immobilized on a flat bottom polystyrene high bind 96-well plate (Costar, 3590) for 16 hours at 4°C, respectively, and then blocked with 100 µl of 4% skim milk (GenomicBase, SKI400) at room temperature for 2 hours. After washed four times with 180 µl of 0.05% PBST, 50 µl of cetuximab Fc variants (CTX-PS101, CTX-PS102, and CTX-PS107) serially diluted with 1% skim milk were dispensed into each well and reacted at room temperature for 1 hour. After washing, antibody reaction was performed for 1 hour at room temperature, and then washed using 50 µl of HRP-Protein L (GenScript, M00098). 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek).

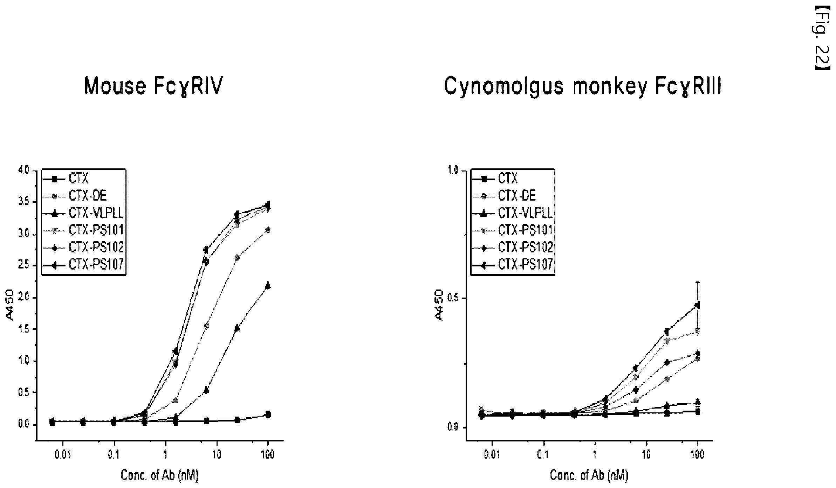

As a result, it was shown that the glycosylated cetuximab Fc variants prepared in the present invention had significantly higher binding affinity than positive controls, glycosylated Fc variants (DE and VLPLL) even in mouse and cynomolgus monkey Fc receptors like human receptors (FIG. 22).

### Example 17. Analysis of binding affinity to Fcγ receptors of glycosylated rituximab Fc variants

### 17-1. Analysis of binding affinity to human FcγRIIIa and FcγRIIb

ELISA assay was performed to confirm the binding affinity to FcyRIIIa and FcγRIIb of the glycosylated rituximab Fc variants (RTX-PS101, RTX-PS102, and RTX-PS107) prepared in Example 15. Specifically, 50 µl of FcγRs-GSTs (FcγRIIIa-158V-GST, FcγRIIIa-158F-GST and FcγRIIb-GST) diluted to 4 µg/ml in 0.05 M Na₂CO₃ pH 9.6 were immobilized on a flat bottom polystyrene high bind 96-well plate (Costar, 3590) for 16 hours at 4°C, respectively, and then blocked with 100 µl of 4% skim milk (GenomicBase, SKI400) at room temperature for 2 hours. After washed four times with 180 µl of 0.05% PBST, 50 µl of glycosylated rituximab Fc variants (RTX-PS101, RTX-PS102, and RTX-PS107) serially diluted with 1% skim milk were dispensed into each well and reacted at room temperature for 1 hour. After washing, antibody reaction was performed for 1 hour at room temperature, and then washed using 50 µl of HRP-Protein L (GenScript, M00098). 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek).

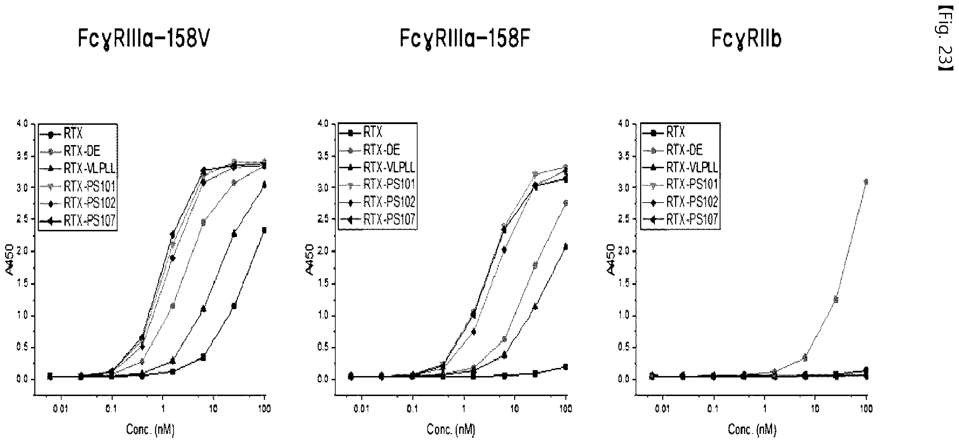

As a result, it was shown that the glycosylated rituximab Fc variants prepared in the present invention had significantly higher FcγRIIIa/ FcγRIIb selective binding affinity than positive controls, glycosylated Fc variants (DE and VLPLL) (FIG. 23). In particular, it was confirmed that significantly excellent FcγRIIIa/ FcγRIIb selective binding properties could be maintained when Fc variants were introduced into different model therapeutic antibodies.

### 17-2. Analysis of binding affinity to mouse FcγRIV and monkey FcγRIII

ELISA assay was performed to confirm the binding affinity to animal Fc receptors corresponding to human FcγRIIIa of rituximab Fc variants. Specifically, 50 µl of mouse FcγRIV-GST and cynomolgus monkey FcγRIII-GST diluted to 4 µg/ml in 0.05 M Na₂CO₃ pH 9.6 were immobilized on a flat bottom polystyrene high bind 96-well plate (Costar, 3590) for 16 hours at 4°C, respectively, and then blocked with 100 µl of 4% skim milk (GenomicBase, SKI400) at room temperature for 2 hours. After washed four times with 180 µl of 0.05% PBST, 50 µl of glycosylated rituximab Fc variants (RTX-PS101, RTX-PS102, and RTX-PS107) serially diluted with 1% skim milk were dispensed into each well and reacted at room temperature for 1 hour. After washing, antibody reaction was performed for 1 hour at room temperature, and then washed using 50 µl of HRP-Protein L (GenScript, M00098). 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek).

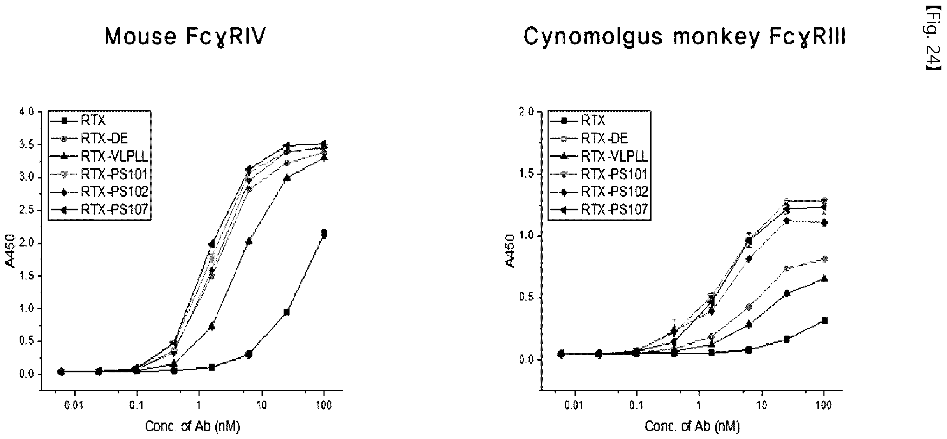

As a result, it was shown that the discovered glycosylated rituximab Fc variants had significantly higher binding affinity than positive controls, glycosylated Fc variants (DE and VLPLL) even in mouse and cynomolgus monkey Fc receptors like human receptors (FIG. 24).

## Claims

1. A human antibody Fc domain variant in which one or more amino acids selected from the group consisting of amino acids at positions 224, 243, 247, 292, 300, 303, 305, 330, 332, 339, 356, and 387 numbered according to a Kabat numbering system in a wide-type human antibody Fc domain are substituted with sequences different from wild-type amino acids.

2. The human antibody Fc domain variant of claim 1, wherein the human antibody Fc domain variant includes one or more amino acid substitutions selected from the group consisting of H224R, F243L, P247I, R292P, Y300L, V303I, V305I, A330L, I332E, A339Q, D356G and P387Q.

3. The human antibody Fc domain variant of claim 1, wherein the human antibody Fc domain variant includes amino acid substitutions of F243L, P247I, R292P, Y300L, V305I, A330L, I332E, A339Q and P387Q.

4. The human antibody Fc domain variant of claim 1, wherein the human antibody Fc domain variant includes amino acid substitutions of H224R, F243L, P247I, R292P, Y300L, V303I, V305I, A330L, I332E, A339Q and P387Q.

5. The human antibody Fc domain variant of claim 1, wherein the human antibody Fc domain variant includes amino acid substitutions of F243L, P247I, R292P, Y300L, V305I, A330L, I332E, A339Q, D356G and P387Q.

6. The human antibody Fc domain variant of claim 1, wherein binding affinity to FcyRIIIa is improved compared to wild-type Fc domains.

7. The human antibody Fc domain variant of claim 1, wherein binding affinity to FcyRIIb is reduced compared to wild-type Fc domains.

8. The human antibody Fc domain variant of claim 1, wherein the human antibody Fc domain variant has improved ability to induce antibody-dependent cell-mediated cytotoxicity (ADCC).

9. An antibody comprising the Fc domain variant of claim 1 or an immunologically active fragment thereof.

10. The antibody or the immunologically active fragment thereof of claim 9, wherein the antibody is a glycosylated antibody.

11. The antibody or the immunologically active fragment thereof of claim 9, wherein the antibody comprises a heavy chain constant region domain 2 (C_{H}2) comprising an amino acid sequence represented by SEQ ID NO: 6 or 7 and a heavy chain constant region domain 3 (C_{H}3) comprising an amino acid sequence represented by SEQ ID NO: 18 or 19.

12. A nucleic acid molecule encoding the Fc domain variant of claim 1, or the antibody or the immunologically active fragment thereof of claim 9.

13. A vector comprising the nucleic acid molecule of claim 12.

14. A host cell comprising the vector of claim 13.

15. A pharmaceutical composition for preventing or treating cancer comprising the Fc domain variant of claim 1, or the antibody or the immunologically active fragment thereof of claim 9 as an active ingredient.

16. The pharmaceutical composition for treating or preventing cancer of claim 15, wherein the cancer is any one or more selected from the group consisting of brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas and pituitary adenomas.

17. A method for preparing a human antibody Fc domain variant with improved selective binding affinity to FcyRIIIa comprising:
a) culturing a host cell comprising a vector comprising a nucleic acid molecule encoding the Fc domain variant of claim 1; and
b) recovering polypeptides expressed by the host cell.

18. A method for preparing a glycosylated antibody with improved selective binding affinity to FcγRIIIa comprising:
a) culturing a host cell comprising a vector containing a nucleic acid molecule encoding the antibody or the immunologically active fragment thereof of claim 9; and
b) purifying antibodies expressed by the host cell.

19. A use of an antibody comprising the Fc domain variant of claim 1 or an immunologically active fragment thereof for use in the preparation of antibody therapeutic agents.

20. A use of an antibody comprising the Fc domain variant of claim 1 or an immunologically active fragment thereof for preventing or treating cancer.

21. A method for treating cancer comprising administering an antibody comprising the Fc domain variant of claim 1 or an immunologically active fragment thereof in a pharmaceutically effective amount to a subject suffering from cancer.
